# EUROPEAN PATENT APPLICATION

(11) **EP 0 867 449 A1**
(43) Date of publication of application: **30.09.1998**
(21) Application number: 96909382.2
(22) Date of filing: 15.04.1996
(51) Int. Cl.: C07K 14/705, C12N 15/12, A61K 38/17, C12P 21/02

(54) **CELL ADHESION PROTEIN, IMMUNOSUPPRESSIVE AGENT CONTAINING THE SAME, AND IMMUNOSUPPRESSIVE AGENT CONTAINING CELLS INDUCED THEREBY**

(30) Priority: 19.04.1995 JP 94060/95; 04.07.1995 JP 169110/95; 27.12.1995 JP 341959/95
(71) Applicant: KANEGAFUCHI KAGAKU KOGYO KABUSHIKI KAISHA, Kita-ku, Osaka-shi, Osaka 530 (JP)
(72) Inventor: YAMASHITA, Kenji, Takasago-shi, Hyogo 676 (JP); NIWA, Hideo, Takasago-shi, Hyogo 676 (JP); OKAZAKI, Takeshi, Kakogawa-shi, Hyogo 675 (JP); FUKUCHI, Takeshi, Akashi-shi, Hyogo 673 (JP); OHARA, Takaaki, Takasago-shi, Hyogo 676 (JP); NISHINO, Tomoko, Himeji-shi, Hyogo 671-02 (JP); KAKUTANI, Tetsu, Kakogawa-shi, Hyogo 675-01 (JP)
(74) Representative: VOSSIUS & PARTNER
(86) International application number: JP9601039
(87) International publication number: WO9633217

(57) **Abstract**

A novel cell adhesion protein having the effect of suppressing various immunological reactions and its analogs; a process for producing the same; an immunosuppressive agent containing the same; and another immunosuppressive agent containing as the active ingredient immunosuppressive cells obtained be incubating human cells in the presence of the cell adhesion protein. These immunosuppressive agents have potent immunosuppressive effects with little side effects.

## Description

### TECHNICAL FIELD

The present invention relates to a novel cell adhesion protein having a suppressive effect on various immunological reactions and an analogue thereof, a method for preparing the same and an iinmunosuppressive agent containing the same, and an immunosuppressive agent which contains as an effective ingredient an immunosuppressive cell obtained by culturing a human cell in the presence of a cell adhesion protein.

### BACKGROUND ART

Generally speaking, an immunosuppressive agent is used for treating disease caused by an abnormal immune hyperfunction, so-called autoimmune disease such as rheumatoid arthritis, systemic lupus erythematosus, chronic nephritis, chronic thyroiditis or autoimmune hemolytic anemia, and for suppressing a rejection reaction in organ transplantation. As an immunosuppressive agent used so far, there are a steroid hormone, azathioprine, cyclophosphamide, and the like.

However, since the immunosuppressive agent used so far acts not only on an immunocyte, but also non selectively on cells in a wide range to influence on their functions and/or growth, serious side effects such as agranulocytosis and nephropathy come into question. Accordingly there has been desired a development of an agent which has a potent immunosuppressive activity and has side effects as little as possible. T cells are cells which play a central role in immunological reactions, and the present inventors have studied to develop a selective agent which targets the T cells.

CD2 molecule is known as a typical one of surface markers which are expressed specifically on T cells. A receptor for CD2 antigen expressed on human T cells (also referred to as T11 antigen) is generally called LFA-3. Heretofore, a gene coding for human LFA-3 has been cloned, and its amino acid sequence has been deduced from its base sequence. See B. P. Wallner et al., J. Exp. Med. vol.166, p.923(1987); B. Seed, Nature vol. 329, p.840(1987). Both LFA-3 and CD2 antigen that is a natural ligand for LFA-3 are cell adhesion proteins participating in immunological reactions such as antigen presentation and activation of killer T cells, and they are classified into the cell adhesion proteins belonging to immunoglobulin superfamily. See A. F. Williams and A. N. Barclay, Annu. Rev. Immunol. vol.6, p.381(1988). A LFA-3 molecule which is generally known at present, is known to be consisted of, from N-terminus, immunoglobulin-like domain 1 (D1 region), immunoglobulin-like domain 2 (D2 region), trans-membrane region that is rich in hydrophobic amino acids (TM region) and comparatively short cytoplasm region (C region). (see J. Exp. Med. vol. 166, p.923, supra.) This molecule is called human TM type LFA-3 in the present specification. Also a human LFA-3 molecule, which has D1 region and D2 region and is bonded to a membrane through glycosyl phosphatidylinositol, is known (see Nature vol.329, p.840, supra). This is called human PI-bonding type LFA-3 in the present specification. And it has been made clear by a prior study of the present inventors that there naturally exists a LFA-3 molecule which has a structure being deficient in D2 region in the above-mentioned human TM type LFA-3 molecule (Japanese Patent Application No. 151792/1991). This molecule is called D2 region-deficient type human LFA-3 like protein in the present specification.

On the other hand, it is known that there exists in a sheep, a CD2 antigen receptor of sheep erythrocytes, which is a protein having a high affinity for CD2 antigen of human T cells. At present, it is understood that the formation of rosette between sheep erythrocytes and human T cells, which is considered as one of reactions specific to human T cells, is a binding reaction caused by a high affinity between CD2 antigen of human T cells and a CD2 antigen receptor of sheep erythrocytes. A structure of a CD2 antigen receptor of sheep erythrocytes has been revealed on the levels of a gene and amino acids through studies done by the present inventors until now (see European Unexamined Patent Publication No. 517174). In the publication, there are cloned three kinds of molecules, that is, a molecule which has, from N-terminus, D1 region, D2 region, TM region and C region, a molecule which has D1 region and D2 region and is bonded to a membrane through glycosyl phosphatidylinositol and a molecule which has D1 region and D2 region and is deficient in TM region and C region (in the present specification, they are called TM type sheep LFA-3, PI type sheep LFA-3 and TM region-deficient type LFA-3, respectively).

These proteins having a high affinity for CD2 antigen of human T cells are considered to be extremely useful as an immunosuppressive agent which has little side effects as mentioned above. However, it is necessary to make mass production possible for this use.

Heretofore, as a method of producing a human CD2 antigen receptor of sheep erythrocytes and an analogue thereof, have been known a method wherein proteins are solubilized from sheep erythrocytes with a surfactant and purified by an affinity chromatography using an antibody therefor (see Japanese Unexamined Patent Publication No. 150228/1988) and a method wherein proteins are solubilized from sheep erythrocytes using trypsin (see T. Kitao, J. Immunol. vol.117, p.310(1976)), and the like. However, they were not suitable for mass production.

The present inventors have previously developed a method for preparing such proteins by genetic engineering techniques, which makes mass production possible. Further it has been confirmed that a LFA-3 molecule derived from a man or sheep, which is deficient in D2 region and TM region and is obtained by genetic engineering techniques using E. coli as a host (in the present specification, they are called human D1HC and sheep D1HC, respectively), has the equivalent ability to inhibit the rosette formation, that is, retains the equivalent affinity for human T cells, in comparison with a LFA-3 molecule derived from a man or sheep, which has D1 region and D2 region and is deficient in TM region and C region and is similarly obtained by genetic engineering techniques using E. coli as a host. Further it has been confirmed that a protein molecule, to which one cysteine residue is added at C-terminus of the above-mentioned human D1HC or sheep D1HC (in the present specification, they are called human D1HCcys protein and sheep D1HCcys protein, respectively) and which has been immobilized onto a carrier, has an affinity for Jurkat cells (see Japanese Unexamined Patent Publication No. 271292/1993). However, a more effective immunosuppressive agent has been desired.

As a preparation example of a dimer or polymer of a LFA-3 molecule, has been known the case where a polymer was obtained by polymerizing a LFA-3 molecule with the aid of phosphatidylinositol group or a sugar chain of the LFA-3 molecule by R. B. Pepinsky et al. (see J. B. C. vol.266, p.18244(1991)), and the case where a dimer of a LFA-3 molecule was obtained by preparing a LFA-3 molecule having a chimera structure with Fc region of an antibody by G. T. Miller et al. (see J. Exp. Med. vol. 178, p.211(1993)). In the former polymer there is observed, a remarkable improvement in the ability to inhibit the rosette formation, which is proportional to a polymerization degree. In the latter dimer, there is also observed an improvement caused by forming a dimer in the ability to inhibit the rosette formation.

However, from the viewpoint of the immunosuppressive ability which is more direct, the former PI-bonding type LFA-3 hardly shows the suppressive activity in MLR. In contrast, although the latter molecule does not have so good ability to inhibit the rosette formation, it shows a superior inhibition activity in MLR and a suppressive system in an antigen-specific activation of T cells. As this suppressive mechanism, G. R. Majeau has given ADCC activity or an injury by bonding the chimera structure of Fc region, which the latter molecule has, to a Fc receptor, which NK cells have (see J. Immunol. vol.176, p.2753(1994)). In such a mechanism, there are expected side effects such as various kinds of infectious disease, which is associated with a hypofunction in a general immune system caused by a non-specific injury of T cells in a living body. And the same thing is expected in an anti-CD2 antibody as to which various kinds of immunosuppressive effects have been reported.

On the other hand, some examples have been known wherein autoimmune disease is caused from quantitative or qualitative abnormality in suppressor T cells (see Mcintosh and Drachman, Science, vol.232, p.401(1986)). A report by Balashov (see Balashov et al., Journal of Clinical Investigation vol.95, p.2711(1995)) has pointed out that there has been observed a remarkable decrease in the efficiency for inducing suppressor T cells by an autologous mixed lymphocyte reaction in patients with MS (multiple sclerosis), and thereby is caused a decrease in the production of γ interferon (IFN) which is a suppressor functional factor of suppressor T cells. Namely, it is thought that, in the patients, cytotoxic cell-inducing T cells and autocytotoxic T cells are induced and activated by a Th activity which is relatively increased by a decrease in the production of a suppressor factor γ IFN caused by an imperfect induction of suppressor T cells. Up to now, several examples of an induction of suppressor T cells have been reported in systems using animals such as a mouse by Fresno et al. (see Fresno et al., Journal of Experimental Medicine, vol.163, p.1246(1980)). Also, as an example of an induction in a man, there is an example performed by using the method wherein an autologous mixed lymphocyte reaction was utilized, which was recently reported in the above-mentioned publication. However, the used method is not desirable at all; because culture needs addition of human serum treated with PHA or ConA, and its operation is complicated, and further there is a dangerous possibility that there occurs contamination of lectin and the like used in a treatment.

### DISCLOSURE OF THE INVENTION

The present invention provides a dimer of an analogue of LFA-3 protein, which has a high affinity for a CD2 antigen of human T cells and exerts a potent immunosuppressive activity in a mechanism with less side effects, a preparation process thereof and an immunosuppressive agent thereof. More particularly, the present invention provides a protein having a structure wherein cysteine residue was introduced at C-terminus or its vicinity of D1 region of human or sheep LFA-3, a method of dimerizing the protein by using SH group of thus introduced cysteine residue, and an immunosuppressive agent containing thus dimerized proteins.

In other words, the present invention relates to a dimer of a protein having an amino acid sequence deficient in the 1st to the 28th amino acids in the amino acid sequence of SEQ ID NO: 1 or an analogue thereof, wherein two of said proteins or analogues are connected each other through SH group of cysteine at C-terminus thereof or its vicinity, a method for preparing the above-mentioned dimer which comprises spontaneously oxidizing the above-mentioned protein or an analogue thereof, and an immunosuppressive agent comprising the above-mentioned dimer. Preferably, the above-mentioned analogue is one into which, from N-terminus, glycine-serine-glycine-serine-serine is inserted just before cysteine at the C-terminus or its vicinity; or one into which, from N-terminus, glycine-serine is inserted just before cysteine at the C-terminus or its vicinity, and from N-terminus, glycine-proline is inserted just after said cysteine.

Also, the present invention provides a DNA coding for a protein which is a soluble D2 region-deficient type human LFA-3 deficient in C region containing one cysteine residue added at C-terminus of said LFA-3 or its vicinity, shown in SEQ ID NO: 7. The above-mentioned soluble D2 region-deficient type human LFA-3 means a human LFA-3 molecule which is deficient in D2 region and TM region and is derived from a man (hereinafter, referred to as human D1HC; and that obtained by deleting C region from the above-mentioned human D1HC and adding one cysteine residue thereto, is referred to as human D1Hcys).

Further, the present invention provides a dimer of a protein having an amino acid sequence deficient in the 1st to the 28th amino acids in the amino acid sequence of SEQ ID NO: 4, wherein two of said proteins are connected each other through SH group of cysteine at C-terminus thereof or its vicinity, a method for preparing the above-mentioned dimer which comprises spontaneously oxidizing the above-mentioned protein, and an immunosuppressive agent comprising the above-mentioned dimer.

Furthermore, the present invention provides a dimer of a protein having the amino acid sequence of SEQ ID NO: 5 or 6, wherein two of said proteins are connected each other through SH group of cysteine at C-terminus thereof or its vicinity, a method for preparing the above-mentioned dimer which comprises spontaneously oxidizing the above-mentioned protein, and an immunosuppressive agent comprising the above-mentioned dimer.

Also, the present invention provides an immunosuppressive agent which comprises as an effective ingredient an immunosuppressive cell which is obtained by culturing a human cell in the presence of a cell adhesion protein. The present invention, by which a cell having an immunosuppressive activity, in particular, an immunosuppressive (suppressor) T cell can be simply produced only with a molecule having a structure extremely similar to molecules which men originally have, provides an extremely important means to treat immune disease, particularly, autoimmune disease and to suppress a rejection reaction in organ transplantation.

The present invention also relates to an iinmunosuppressive agent which comprises as an effective ingredient a cell adhesion protein.

In the above-mentioned immunosuppressive agent, the following cases are preferable;
the case where the above-mentioned cell adhesion protein is a dimer of a protein having the amino acid sequence of SEQ ID NO: 1, or a derivative thereof, wherein two of the above-mentioned proteins or derivatives are connected each other through SH group of cysteine at C-terminus thereof or its vicinity,
the case where the above-mentioned cell adhesion protein is a dimer of a protein which has an amino acid sequence deficient in the 1st to the 28th amino acids in the amino acid sequence of SEQ ID NO: 1 and/or
into which glycine-serine-glycine-serine-serine is inserted just before cysteine at the C-terminus, or into which glycine-serine-serine is inserted just before cysteine at the C-terminus, and glycine-proline is inserted just after the above-mentioned cysteine, wherein two of the above-mentioned proteins are connected each other through SH group of cysteine at the C-terminus or its vicinity,
the case where the above-mentioned cell adhesion protein is a dimer of a protein having the amino acid sequence of SEQ ID NO: 4, or a dimer of a derivative having an amino acid sequence deficient in the 1st to the 28th amino acids in the amino acid sequence of SEQ ID NO: 4, wherein two of the above-mentioned proteins or derivatives are connected each other through SH group of cysteine at C-terminus thereof or its vicinity, the case where the above-mentioned cell adhesion protein is a dimer of a protein having the amino acid sequence of SEQ ID NO: 5 or 6, wherein two of the above-mentioned proteins are connected each other through SH group of cysteine at C-terminus thereof or its vicinity,
the case where the above-mentioned human cell is a human peripheral blood cell, and the above-mentioned immunosuppressive cell is obtained by culturing the human peripheral blood cell in the presence of 1L-2 and or GM-CSF together with a cell adhesion protein,
the case where the above-mentioned immunosuppressive cell is obtained by culturing a medium containing serum, and the case where the above-mentioned serum is autologous serum.

### BRIEF EXPLANATION OF DRAWINGS

Fig. 1 is a graph showing a making procedure of pβLFA3-1.

Fig. 2 is a graph showing a making procedure of pSVLFA3-1.

Fig. 3 is a graph which shows the measured absorbance in the performed ELISA assay with respect to a serum-free conditioned medium of a transformant of pβLFA3-1 (Fig. 3A) or pSVLFA3-1 (Fig. 3B) obtained in Example 1, by using a natural type LFA-3 molecule which was purified from JY cells as a positive control and as a temporary standard and by using a serum-free conditioned medium of a transformant of pSV2dhfr as a control.

Fig. 4 is a figure in which the amino acid sequence of human LFA-3 is compared with the amino acid sequence of sheep ΔD2 protein. Number in the figure is number of an amino acid from N-terminus of human LFA-3. A symbol "―" represents a deficient amino acid which does not exist in one sequence. Amino acids which are homologous in both sequences are shown with a colon. A sequence from the 94th to the 181th, which is underlined, is D2 region, and sheep ΔD2 protein does not have a sequence of this underlined part.

Fig. 5 is a graph showing a making procedure of pβLT1dhfr.

Fig. 6 is a graph showing results of ³H-thymidine incorporation into human peripheral blood cells, which were measured by adding PBS, a monomer of human D1Hcys protein and a dimer of human D1Hcys protein or sheep D1HCcys protein with PPD antigen.

Fig. 7 shows a pattern of a flow cytometry by using a fluorescent CD3 antibody after culturing human peripheral blood cells with added PBS.

Fig. 8 shows a pattern of a flow cytometry by using a fluorescent CD3 antibody after culturing human peripheral blood cells with an added dimer of human D1Hcys protein.

Fig. 9 shows a pattern of a flow cytometry by using a fluorescent CD3 antibody after culturing human peripheral blood cells with an added monomer of human D1Hcys protein.

Fig. 10 is a graph showing an immunosuppressive activity of human T cells treated with a dimer of human D1Hcys or sheep D1HCcys.

Fig. 11 is a graph showing an immunosuppressive activity of human peripheral blood cells treated with human D1Hcys, IL-2 and GM-CSF, and a combination thereof.

### BEST MODE FOR CARRYING OUT THE INVENTION

The protein of the present invention is a dimer of human D1Hcys protein or an analogue thereof, or a dimer of sheep D1HCcys protein, wherein two of the above-mentioned proteins or analogues are connected each other through SH group of cysteine at the C-terminus or its vicinity. The amino acid sequence of human D1Hcys protein produced in a cultured animal cell and the base sequence thereof are shown in SEQ ID NO: 1 and NO: 7, respectively. Also, the amino acid sequence of sheep D1HCcys protein produced in a cultured animal cell and the base sequence thereof are shown in SEQ ID NO: 4 and NO: 10, respectively. However, human D1Hcys and sheep D1HCcys are naturally cleaved between the 28th amino acid and the 29th amino acid, when they are secreted outside the cell.

Herein the term analogue means a protein which is obtained by adding and/or inserting and/or substituting and/or deleting one or plural amino acids in an amino acid sequence of an original protein, and is the same in essence as the original protein. Concrete example of the analogue of human D1Hcys protein is a protein having an amino acid sequence deficient in the 1st to the 28th amino acids in the amino acid sequence of SEQ ID NO: 1 into which, from N-terminus, glycine-serine-glycine-serine-serine is inserted just before cysteine at the C-terminus or its vicinity; or into which, from N-terminus, glycine-serine-serine is inserted just before cysteine at the C-terminus or its vicinity, and, from N-terminus, glycine-proline is inserted just after the above-mentioned cysteine. As each concrete example, the amino acid sequences are shown in SEQ ID NO: 2 and 3, and the base sequences are shown in SEQ ID NO: 8 and 9, respectively. In the case where, from N-terminus, glycine-serine-glycine-serine-serine is inserted just before cysteine at the C-terminus or its vicinity, because the hydrophobic amino acids are introduced, it is expected that forming efficiency of a dimer is improved or that binding ability is improved through a reduction of steric hindrance in a molecule after the dimer formation. And in the case where glycine-serine-serine and glycine-proline are inserted before and after cysteine at the C-terminus or its vicinity, it is expected to bring about a preventing effect on decomposition and elimination of cysteine residue at the C-terminus or its vicinity caused by a protease. And as an example of sheep D1HCcys protein produced in E. coli, the amino acid sequences are shown in SEQ ID NO: 5 and 6, and the base sequences are shown in SEQ ID NO: 11 and 12, respectively.

Monomers of these proteins may be ones obtained from natural materials by extraction and purification, and also the monomers can be obtained by a method of gene recombination as shown in Examples of the present application. And these proteins can be synthesized by a chemical method on the basis of the base sequences and/or the amino acid sequences shown in SEQUENCE LISTING related to the present invention.

The dimer of the present invention can be obtained by spontaneously oxidizing thus obtained monomer, that is, human D1Hcys protein or an analogue thereof or sheep D1HCcys protein. The spontaneous oxidation can be performed, for example, by dialyzing a monomer of a protein dissolved in a buffer which contains a reducing agent against a buffer which contains no reducing agent. Concretely the spontaneous oxidation can be performed by putting a monomer of a protein in a dialysis membrane, and dialyzing it overnight at 25° to 30°C against 50 mM Tris-hydrochloric acid buffer, 2mM EDTA (pH 8.5). It is also possible to produce various kinds of polymers similarly by utilizing SH group.

The immunosuppressive agent of the present invention contains the above-mentioned protein dimer according to the present invention as an effective ingredient. Other ingredients necessary for formulation can be contained optionally. Examples of such ingredients are, for instance, distilled water for an injection, sodium chloride, phosphoric acid, human serum albumin, calcium salt, magnesium salt, and the like. The administration route of the immunosuppressive agent of the present invention is not limited. However, it is preferable to administer it systemically or locally as an injection.

The immunosuppressive agent of the present invention has a superior immunosuppressive effect and little side effects. Accordingly the immunosuppressive agent of the invention is effective in treating disease caused by an abnormal immune hyperfunction, treating autoimmune disease such as rheumatoid arthritis, systemic lupus erythematosus, chronic nephritis, chronic thyroiditis or autoimmune hemolytic anemia, suppressing a rejection reaction in organ transplantation, and further treating allergic disease, and the like. An amount of the dosage varies in dependence on a purpose of administration, that is, a kind of disease and a degree of a symptom of a patient to whom it is administered. However, generally speaking it is 1 to 100 mg/day, preferably 1 to 10 mg/day, and the most preferably 1 mg/day for an adult.

Also, there is explained the immunosuppressive agent of the present invention, which is obtained by culturing a human cell in the presence of a cell adhesion protein.

The term human cell means a cell constructing a human body or a cell derived therefrom.

Examples of the human cell used in the present invention are, for instance, a human peripheral blood cell, a human peritoneal cell, a human thymocyte, a human myelocyte, a human splenocyte, a human T cell line, and the like. In the case where a patient is actually treated by a method of cell transfer, it is the most desirable to use a cell of a patient himself from the viewpoint of an immune rejection reaction or an immunosuppressing effect. Among them, a human peripheral blood cell is preferable from the viewpoint of easy collection. A human peripheral blood cell can be obtained by laying venous blood obtained by drawing blood from a man, on 50 mℓ of Ficoll in a polystyrene tube (Ficoll-Paque, made by Pharmacia), centrifuging the tube for 20 minutes at 1500 r.p.m. to separate a fraction containing a lymphocyte and a monocyte which is formed on a boundary surface, and washing the fraction three times with RPMI-1640 culture medium (Ficoll centrifugal separation method). A T cell can be obtained by adding thus obtained human peripheral blood cell to a culture medium and culturing it overnight to collect a suspending cell.

The term cell adhesion protein, which is used in culturing a human cell or is contained in the immunosuppressive agent as an effective ingredient, means a protein having a function for mediating adhesion between cells. Examples of the cell adhesion protein are, for instance, immunoglobulin superfamily, integrin superfamily, cadherin, and the like. Immunoglobulin superfamily is preferable, and LFA-3 protein having a high affinity for CD2 antigen of a human T cell and an analogues thereof are particularly preferable. As more preferable examples, there are given a dimer of human D1HC protein or its derivative, and a dimer of sheep D1HCcys protein or its derivative, which are analogues of LFA-3 protein, especially the dimer of the protein according to the present invention. Hereinafter these proteins are explained.

The amino acid sequence of human LFA-3 protein is shown in SEQ ID NO: 22. Human LFA-3 protein is consisted of immunoglobulin-like domain 1 (D1 region), immunoglobulin-like domain 2 (D2 region), trans-membrane region (TM region) and cytoplasm region (C region) (see B. P. Wallner et al., Journal of Experimental Medicine vol.166, p.923(1987)). Human D1HC is deficient in D2 region and TM region in human LFA-3 protein, and human D1Hcys is obtained by further deleting C region from human D1HC and adding one cysteine residue at its C-terminus. The position where one cysteine residue is added is not only C-terminus but also its vicinity. The amino acid sequence of human D1Hcys produced by a cultured animal cell is shown in SEQ ID NO: 1, and the base sequence of DNA which codes for this protein is shown in SEQ ID NO: 7. However, human D1Hcys is spontaneously cleaved between the 28th amino acid and the 29th amino acid, when it is secreted outside the cell. Accordingly, in being secreted outside the cell, human D1Hcys has a sequence deficient in the 1st to the 28th amino acids in the amino acid sequence of SEQ ID NO: 1. The dimer of the protein according to the present invention is the same as the protein explained above.

The amino acid sequence of sheep D1HCcys produced by a cultured animal cell is shown in SEQ ID NO: 4, and the base sequence of DNA which codes for this protein is shown in SEQ ID NO: 10. However, sheep D1HCcys is spontaneously cleaved between the 28th amino acid and the 29th amino acid, when it is secreted outside the cell. Accordingly, in being secreted outside the cell, sheep D1HCcys has a sequence deficient in the 1st to the 28th amino acids in the amino acid sequence of SEQ ID NO:4.

These proteins may be ones obtained from natural materials by extraction and purification, and also these can be obtained by a method of gene recombination as shown in Examples of the present application. And these proteins can be synthesized by a chemical method on the basis of the base sequences and/or the amino acid sequences shown in the SEQUENCE LISTING.

A preferable dimer of a protein as a cell adhesion protein used in the present invention is formed by connecting the above-mentioned protein monomers each other through SH group of cysteine residue at the C-terminus or its vicinity. Such a protein dimer can be obtained by spontaneously oxidizing thus obtained protein monomer. The spontaneous oxidation can be performed, for example, by dialyzing a monomer of a protein dissolved in a buffer which contains a reducing agent against a buffer which contains no reducing agent.

The term immunosuppressive cell means a cell having the ability to suppress an immune response. The activity for suppressing an immune response can be measured by using a reaction system such as one obtained by mixing peripheral blood cells which are prepared from different two or more individuals (MLR) or one obtained by mixing a certain antigen and a peripheral blood cell of an individual having a T cell, which has a reactivity the antigen (memory T cell). As explained in the following Test Example 2, the suppressive activity can be measured by adding a cell which may have an immunosuppressive activity to a human peripheral blood cell (hereinafter referred to as PBL) which is freshly obtained from the same man in vitro, further adding an antigen such as PPD or TT (tetanus toxoid), culturing the cells far several days under the conditions of 37°C and 5 % CO₂, adding [ ³H]thymidine thereto, further culturing the cells for 5 to 16 hours and measuring radioactivity in the cells by a scintillation counter.

The immunosuppressive cell can be obtained by culturing a human cell in the presence of the above-mentioned cell adhesion protein. An amount of the cell adhesion protein added to a culture medium varies in dependence on a kind of the used protein, and there is no limitation particularly. However, 0.1 to 10 µg/mℓ is preferable. In case of too little, it is feared that an immunosuppressive activity is not induced. In contrast, in case of too much, there is the possibility that a cell is injured. Examples of the culture medium which can be used are, for instance, a culture medium for culturing an animal cell, D-MEM, RPMI-1640 and the like. A lymphokine such as IL-2 or GM-CSF may be added alone or in admixture thereof to such a culture medium. Generally speaking, compared with a cell cultured with only a cell adhesion protein, a cell having a more potent immunosuppressive activity can he obtained by further adding such a lymphokine. An amount of the added lymphokine is not particularly limited. However, it is preferable to add 0 to 100 ng/mℓ of GM-CSF and to add 0 to 500 units/mℓ of IL-2. In case of too much, there is also the possibility that a cell is injured. Also serum may be added to a culture medium. Autologous serum is preferably used. When autologous serum is used, cell growth is good, and a non-specific immune reaction is difficult to occur. Therefore, autologous serum is the most desirable. An amount of the added serum is not limited in particular. However, 2 to 10 % is general. In case of too much, a cell is injured, and there occurs a decrease in proliferation potency or a decrease in inducing of a suppressive function. The immunosuppressive cell is obtained by culturing a human cell in such a culture medium normally for 1 to 7 days under the conditions of 37°C and 5 % CO₂. The term autologous serum means serum which is obtained from an individual from which is obtained a human cell to he used for a culture.

The immunosuppressive agent of the present invention contains the above-mentioned immunosuppressive cell as an effective ingredient. Other ingredients necessary for formulation can be optionally contained. Examples of such ingredient are, for instance, distilled water for an injection, sodium chloride, phosphoric acid, human serum albumin, calcium salt, magnesium salt and the like. The administration route of the immunosuppressive agent according to the present invention is not limited. However, it is preferable to administer it systemically or locally using a medical care equipment such as a catheter.

The immunosuppressive agent of the present invention has a superior immunosuppressive activity and further has little side effects. Accordingly it is effective for treating disease caused by an abnormal immune hyperfunction, treating autoimmune disease such as rheumatoid arthritis, systemic lupus erythematosus, chronic nephritis, chronic thyroiditis or autoimmune hemolytic anemia, suppressing a rejection reaction in organ transplantation, and further treating allergic disease, and the like.

An amount of the dosage varies in dependence on a purpose of administration, that is, a kind of disease and a degree of a symptom of a patient to whom it is administered. However, in case of a treatment wherein a cell treated outside a body is added to the body, the cell number is 10⁶ to 5 × 10⁸, preferably 10⁶ to 10⁸, more preferably 10⁶ for an adult per day.

And in case of a treatment by administering the immunosuppressive agent containing a cell adhesion protein as an effective ingredient, an amount of the dosage, generally speaking, is 1 to 100 mg/day, preferably 1 to 10 mg/day, and more preferably 1 mg/day for an adult.

### EXAMPLES

Hereinafter, the present invention is more specifically explained by means of the following Examples. However, the present invention is not limited to only the Examples.

### EXAMPLE 1

### Prepartion of dimer of human D1Hcys protein

### ① Production of monomer of human D1Hcys protein in cultured animal cells

### a) Separation of transformants of cultured animal cells which produce human D1Hcys protein

### (Making of expression vector)

### Making of pβLFA3-1:

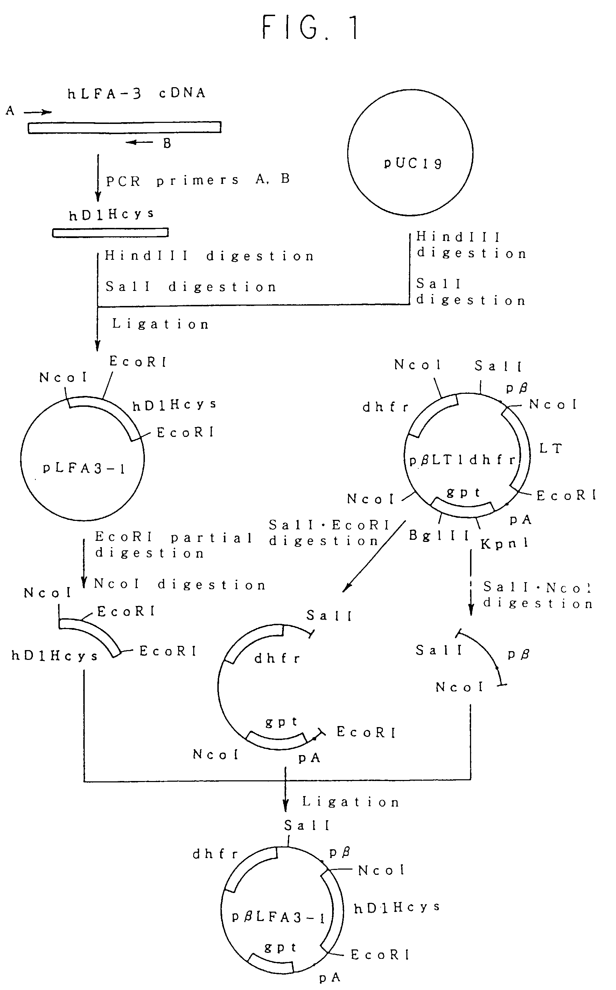

Using polyA(+)RNA which was prepared from the human T cell line MOLT-4 (ATCC No. CRL-1582), a cDNA coding for human LFA-3 (hLFA-3) was made as shown in Fig. 1. For this cDNA, PCR (Polymerase Chain Reaction) was performed with primers A and B which are shown in SEQ ID NO: 13 and SEQ ID NO: 14, respectively to give a cDNA (SEQ ID NO: 7) coding for LFA-3 deficient in D2 region, TM region and C region to which one cysteine residue was added at the C-terminus, i.e., hD1Hcys protein. After having cleaved thus obtained cDNA with restriction enzymes HindIII and SalI, the cleaved cDNA was connected with plasmid pUC19 (made by Takara Shuzo Co., Ltd.) which was obtained by cleaving identically with restriction enzymes HindIII and SalI to give plasmid pLFA3-1. Then, this plasmid was partially digested with EcoRI, followed by cleaving with NcoI to recover a fragment for the above-mentioned LFA-3.

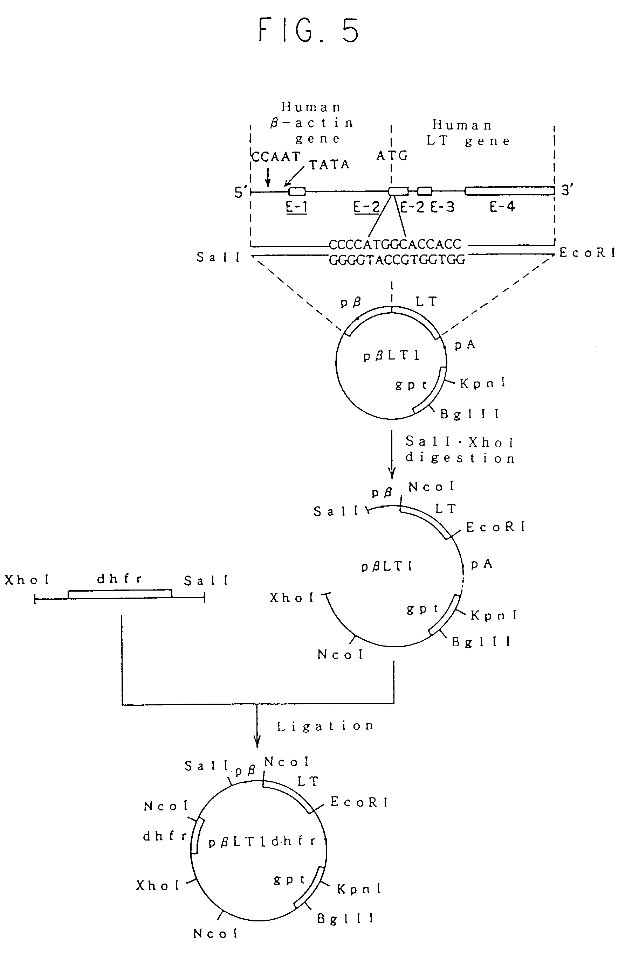

Plasmid pβLT1dhfr was prepared as follows. The structure of plasmid pβLT1dhfr being a human LT expression vector is shown in Fig. 5. In construction of the expression vector, NcoI recognition site was introduced at an initiation site of a human LT gene, and a SalI-NcoI fragment (1.2Kb) of a human β-actin gene was connected with the human LT gene at the initiation site (Fig. 5). As mentioned later, there were used all useful sequences of a 5'-untranslated region and sequences adjacent to 5' side which include an intervening sequence (intron) 1 of the human β-actin gene (about 1.2 kb). In such a manner, construction of plasmid pβLT1 and preparation of DNA were performed, and all plasmids were amplified in E. coli HB101. The pβLT1 contains a human β-actin gene promoter at 5' end and a human genome LT gene flanked with a polyadenylated sequence of SV40 at 3' end. As a selectable marker, the pβLT1 has a bacterial gpt gene connected to an early promoter of SV40. Then, the pβLT1dhfr was constructed by cloning a XhoI/SalI fragment, which contains a cDNA gene for mouse dhfr under the transcriptional control of the major late promoter of an adenovirus, at SalI recognition site of the pβLT1 (see Agricultural and Biological Chemistry No.2, vol.55, p501(1991) by Nakagawa et al.).

The fragment which contained the selectable marker gene was recovered after digesting thus obtained pβLT1dhfr with SalI and EcoRI. Also the fragment which contained the promoter was recovered after digesting the pβLT1dhfr with SalI and NcoI. The objective expression vector pβLFA3-1 was obtained by connecting these two fragments and the fragment for the above-mentioned LFA-3 with the aid of DNA ligase, and transforming E. coli JM109 thereby (Fig. 1).

### Making of pSVLFA3-1:

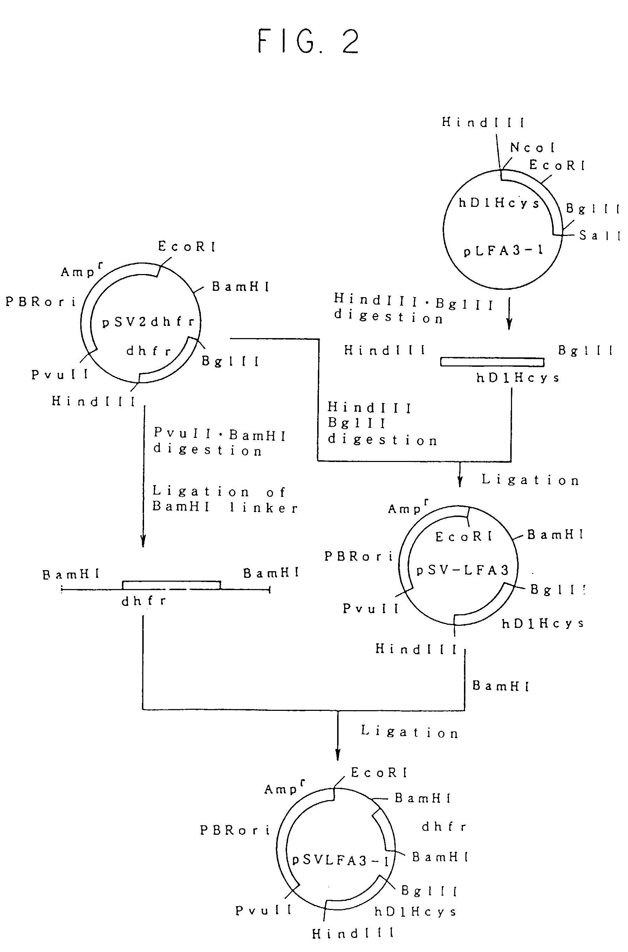

As shown in Fig. 2, a fragment for a LFA-3 (hD1Hcys) was obtained by cleaving the above-mentioned vector pLFA3-1 with restriction enzymes HindIII and BglII. Then, a fragment which contained a selectable marker was obtained by similarly cleaving pSV2dhfr (ATCC No. 37146) with HindIII and BglII. Plasmid pSV-LFA3 was prepared by connecting these fragments. Expression vector pSVLFA3-1 was prepared by inserting the fragment, which was obtained by cleaving the pSV2dhfr with PvuII and BamHI and adding BamHI linkers, into the position cleaved with restriction enzyme BamHI of the vector pSV-LFA3.

### (Introduction of DNA into cell and selection of transformed strain)

Transfection was performed by adding 250 µℓ of a solution containing thus obtained vector pβLFA3-1 or pSVLFA3-1 (1 µg) and 0.25M of CaCl₂, to HEPES buffered saline (280 mM NaCl, 1.5 mM Na₂PO₄, 50 mM HEPES, pH 7.1), leaving thus obtained solution at room temperature for 30 minutes and then adding the solution to cultured cells (a concentration of 2 × 10⁵/mℓ in dissemination) which were growing in a plastic culture plate of a diameter of 100 mm. As cultured cells, there were used CHO-K1 (ATCC No. CRL9618), CHO-DG44 (obtained from Chasin, professor at Columbia University, U.S.A.), CHO-DHFR (-)-DUX-B11 (obtained from Chasin, professor at Columbia University, U.S.A.) and BHK (ATCC No. CRL8544) cells, and 10 mℓ of a culture medium suitable for each host cell was used as a culture medium in the culture plate. That is, Dulbecco modified MEM culture medium supplemented with 5 % bovine serum, 100 µg/mℓ of penicillin and 100 µg/mℓ of streptomycin (D-MEM made by NISSUI CO., LTD.) was used for CHO-K1 and BHK cells, and alpha MEM culture medium (including a nucleic acid) supplemented with the same components as mentioned above was used for CHO-DG44 and CHO-DHFR(-)-DUX-B11 cells.

After 3 to 16 hours of transfection, a culture medium was removed, and cells were washed with phosphate buffered saline (PBS, pH7.2). The cells were cultured further for two days at 37°C by adding the culture medium of the same composition to the culture plate.

Thereafter the culture medium was changed with a flesh culture medium which contained a selectable reagent, and culture was continued. As the culture medium which contained a selectable reagent, D-MEM culture medium which contained 25 µg/mℓ of mycophenolic acid, 25 µg/mℓ of adenine, 200 µg/mℓ of xanthine, 5 µg/mℓ of thymidine and 0.1 µg/mℓ of aminopterin was used for CHO-K1 and BHK cells, and nucleic acid-free alpha MEM culture medium which contained 5 % dialyzed bovine serum was used for CHO-DG44 and CHO-DHFR(-)-DUX-B11 cells. Colonies from the cells, in which plasmids were taken and the mycophenolic acid-resistant gene gpt or the dhfr gene was expressed, were observed in about 2 weeks. The transformed cells which produced human D1Hcys protein were identified by ELISA method with monoclonal antibody TS2/9. The above-mentioned TS2/9 was obtained by growing hybridoma HB205 (ATCC No. HB205) in a mouse abdominal cavity and purifying the antibody TS2/9 in the ascites by means of a column in which protein A was immobilized to agarose gel (Affi-gel Protein A made by BIO RAD company). Concrete purification method comprised mixing the conditioned medium of the hybridoma with an equivalent amount of a binding buffer (pH 9.0) (made by BIO RAD company) and applying the mixture to the above-mentioned column. After washing with an amount being 15 times the column volume of the above-mentioned binding buffer, the above-mentioned TS2/9 was eluted using an elution buffer (pH 3.0) (made by BIO RAD company). The eluted liquid was immediately neutralized with 1M Tris-hydrochloric acid (pH 9.0).

### b) Identification of cell line producing human D1Hcys protein by ELISA method

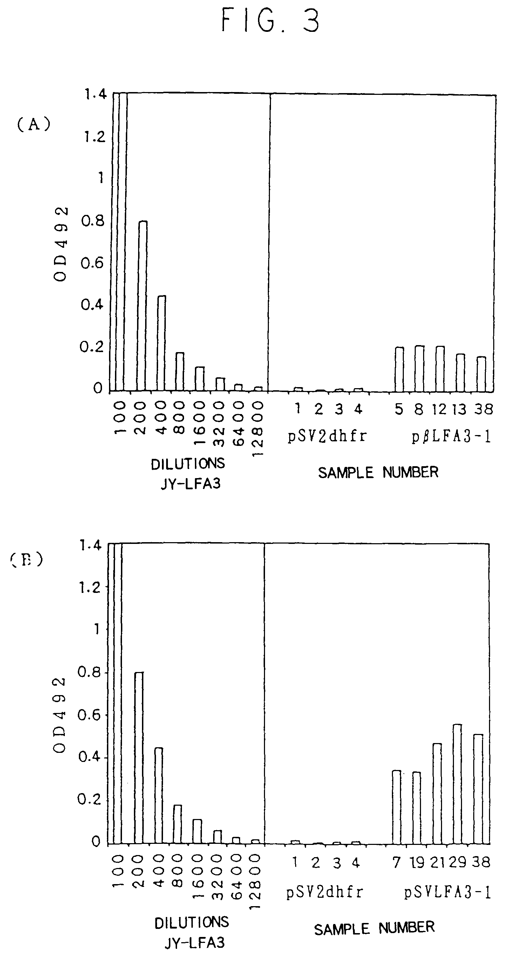

In each microtiter well, 50 µℓ of a serum-free conditioned medium of the above-mentioned transformed cells was put, and the well was left for 12 to 16 hours at 4°C . After adsorption, the medium was removed from the well with an aspirator. Then, the well was washed twice with PBS containing 0.05 % Tween 20, and thereto was added 50 µℓ of a 1 % gelatin/PBS solution to incubate the well for one hour at 37°C. After the incubation, the well was washed with PBS containing 0.05 % Tween 20, and thereto was added 50 µℓ of a 1 µg/ml TS2/9 solution (PBS) to incubate the well for one hour at 37°C . Thereafter, the well was washed three times with PBS containing 0.05 % Tween 20, and thereto was added 50 µℓ of a rabbit anti-mouse IgG (in PBS) which was labeled with a horseradish peroxidase to incubate the well for one hour at 37°C. After washing the well five times with PBS containing 0.05 % Tween 20, to each well was added 100 µℓ of a solution prepared by adding 4 µℓ of a 30 % H₂O₂ aqueous solution to 10 mℓ of citrate phosphate buffer (pH 5) containing 0.1 mg/mℓ orthophenylenediamine. The well was incubated for 30 minutes and, the reaction was terminated by adding 100 µℓ of 2.5M sulfuric acid thereto. Then absorbance at 492 nm was measured with a multi scan (made by Titertech company). The results of assay for transformed cells with the above-mentioned pβLFA3-1 or pSVLFA3-1 were shown in Fig. 3. As a major positive control and temporary standard was used a natural type LFA-3 molecule, which was purified from JY cells using the method of M. L. Dustin et al. (see Journal of Experimental Medicine vol.165, 677(1987)). As a control group, several cell lines, which were transformed with the above-mentioned pSV2dhfr, were subjected to ELISA assay.

### c) Purification of human D1Hcys protein produced by cultured animal cells by antibody-immobilized column

The mouse cell line HB205 (ATCC No. HB205) which secretes monoclonal antibody TS2/9 was grown in a mouse abdominal cavity, and the antibody TS2/9 in the ascites was purified by a protein A-immobilized column in the same manner as described above. Then, the eluate from the column was coupled to AffiGel 10 affinity column in a concentration of 3 mg/mℓ (resin), to which N-hydroxysuccinimide ester was immobilized as a ligand (made by BIO RAD company). This column (5 mℓ) was equilibrated successively with PBS, a 50 mM elution buffer of glycine - HCl (pH 3.0), 0.25M NaCl and finally PBS.

A serum-free conditioned medium of a cell line judged positive by ELISA in the above-mentioned b) was applied to the above-mentioned column at the rate of 40 mℓ/hr. After washing sufficiently with PBS, a substance specifically bonded thereto was eluted with the above-mentioned elution buffer, immediately the eluted liquid was neutralized with an equivalent amount of 1M Tris-HCl (pH 8.5) to adjust a pH to neutrality, and stored at 4°C.

### ② Making of dimer of human D1Hcys protein

To 5 mℓ of a fraction containing human D1Hcys protein obtained in the above-mentioned ① (1 mg/mℓ) (in a mixture of 50mM glycine-HCl (pH 3.0) and 1M Tris-hydrochloric acid (pH 8.5) in the equivalent mixing ratio), was added 80 mℓ of a solution containing 50 mM Tris-hydrochloric acid, 2mM EDTA and 50 mM 2-mercaptoethanol to give a whole amount of 85 mℓ. Thus obtained liquid was dialyzed overnight against 5L of a solution containing 50 mM Tris-hydrochloric acid and 2 mM EDTA (pH 8.5). During this process, the dimer was gradually formed. After the dialyzed liquid was concentrated using an ultrafiltration membrane of cut-off molecular weight 3000, this concentrated liquid was applied to gel filtration column Sephacryl S-200 (2.2 cm diameter, 320 mℓ) (purchased from Pharmacia), which was equilibrated with 50 mM Tris-hydrochloric acid, 2 mM EDTA (pH 8.5), at the flow rate of 57 mℓ /hr. The dimer and the monomer of human D1Hcys protein were separated by feeding the above-mentioned buffer at the same flow rate. The yield was 670 µg as a whole.

### EXAMPLE 2

### Preparation of dimer of sheep D1HCcys protein

### ① Making of monomer of sheep D1HCcys protein in E. coli

### a. Synthesis of PCR primers for cDNA coding for solubilized D2 region-deficient type sheep LFA-3 protein (hereinafter referred to sheep ΔD2 protein)

As PCR primers coding for cDNA for sheep ΔD2 protein, mixed primers shown in SEQ ID NO: 16, which had a sequence containing the recognition sequence of restriction enzyme BamHI and a following DNA sequence deduced from the sequence of the 1st-7th amino acids of LFA-3 shown in SEQ ID NO: 15; and a primer shown in SEQ ID NO: 17, which had a sequence containing the recognition sequence of restriction enzyme PstI and a following DNA sequence deduced from the sequence of the 22th-27th amino acids shown in SEQ ID NO: 15 were synthesized by means of a DNA synthesizer (Model 381A, made by Applied Biosystems).

From a sheep, 100 mℓ of blood was collected with heparin, and a buffy coat fraction was obtained by centrifuging the collected blood at 350G for 10 minutes. After lysis of erythrocytes in the fraction using an erythrocyte lysing buffer, lysate was washed twice with an isotonic phosphate buffer to give sheep leukocytes. Then, from the obtained sheep leukocytes was extracted RNA by guanidine thiocyanate method, and further polyA(+)RNA was purified by means of an oligo(dT)-cellulose column (see Rabomanyuaruidenshikogaku, edited by Masami Muramatsu, Maruzen (1988)). Double stranded cDNA was synthesized from the polyA(+)RNA by means of a commercially available kit (You-Prime cDNA Synthesis Kit, made by Pharmacia).

### c. Amplification by PCR method and cloning of DNA sequence coding for sheep ΔD2 protein

By PCR method was amplified a cDNA coding for sheep ΔD2 protein in vitro. That is, using 100 µℓ of a reaction mixture containing 10 mM Tris-hydrochloric acid (pH 8.3), 100 mM potassium chloride, 1.5 mM magnesium chloride, 0.01 % gelatin, 10 µM of each of 2 kinds of the mixed primers synthesized in the above-mentioned a, 10 ng of the cDNA of the sheep leukocytes prepared in the above-mentioned b, 0.2 mM of 4 kinds of deoxyribonucleic triphosphates and 2.5 units of Taq DNA polymerase, 35 cycles of PCR were carried out under the reaction conditions of 94°C, 1 minute; 37°C , 2 minutes; and 72°C 2 minutes. After completing the reactions, size of PCR products was measured by a polyacrylamide gel electrophoresis, and then it was found that the DNA of about 100 base pairs was amplified. The DNA of about 100 base pairs was extracted from the gel and treated with restriction enzymes BamHI and PstI. Then the treated DNA was inserted into BamHI-PstI site of M13mp19 phage vector (made by TAKARA SHUZO Co.) and cloned using E. coli JM109 as a host.

### d. Sequencing of gene amplified by PCR

There was determined a sequence of the DNA of about 100 base pairs prepared from a positive clone obtained in the above-mentioned c by a conventional method using dideoxynucleotide triphosphates. As a result, the sequence between 2 kinds of the primers used in PCR was determined to be the sequence of the 19th-65th bases in the DNA sequence of SEQ ID NO: 18. The determined sequence corresponds to the sequence from the 7th amino acid (Gly) to the 22nd amino acid (Pro) of the amino acid sequence of sheep ΔD2 protein, which was determined later, shown in SEQ ID NO: 19. It is shown that the cDNA of sheep ΔD2 protein contains the above-mentioned base sequence. The DNA sequence codes the 7th-22nd amino acids in SEQ ID NO: 19.

### e. Isolation of cDNA of sheep ΔD2 protein

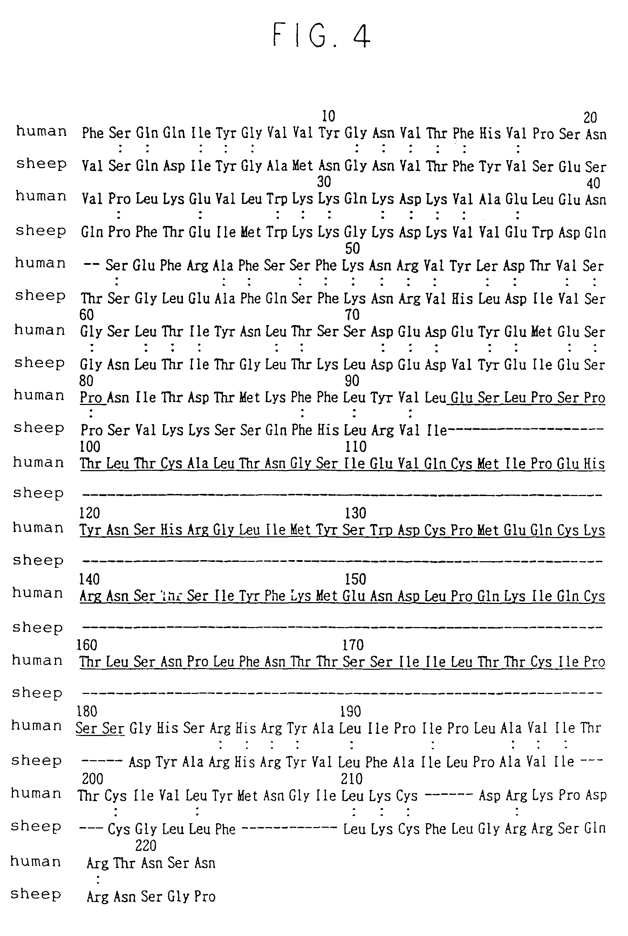

In order to clone a full-length cDNA of sheep ΔD2 protein, sheep cDNA libraries were screened using the N-terminal cDNA sequence of sheep ΔD2 protein determined in the above-mentioned d as a probe. That is, the double stranded cDNA derived from sheep leukocytes prepared in the above-mentioned b was treated with DNA polymerase to give a DNA fragment with blunt ends to which EcoRI linkers (made by Pharmacia) were connected. Further, the linked DNA fragment was cut with EcoRI, and thereto were connected right and left arms of λgt11 (made by Stratagene) treated with alkaline phosphatase. Packaging in vitro was carried out to give cDNA libraries. There were synthesized probes having a cDNA sequence adjacent N-terminus of sheep ΔD2 protein, i.e., the probe having the sequence of SEQ ID NO: 20 and that of SEQ ID NO: 21. About 2 × 10⁵ recombinant phages were screened using these probes. As a result, 3 positive clones (SL-6, SL-40 and SL-43) containing 1.0 kb (kilobase)-1.2 kb of the inserted cDNA were obtained. Among the positive clones, the cDNA sequence of SL-40 was determined by dideoxy method using M13 phage. As a result, it is revealed that sheep ΔD2 protein is encoded by the base sequence of SEQ ID NO: 18 and has the amino acid sequence of SEQ ID NO: 19. It is also revealed that sheep ΔD2 protein has a signal peptide having 28 amino acid residues which starts from methionine (Met). On the basis of thus obtained information of the cDNA base sequence of sheep ΔD2 protein, a natural type sheep ΔD2 protein and a derivative thereof can he produced by genetic engineering techniques. The amino acid sequence of SEQ ID NO: 22 is that of human LFA-3 which has been reported by Wallner et al. (refer to B. P. Wallner et al., Journal of Experimental Medicine, vol.166, p.923(1987)), and SEQ ID NO: 28 shows the DNA sequence thereof. Also, Fig. 4 shows correspondence of the amino acid sequence of sheep ΔD2 protein shown in SEQ ID NO: 19 to that of human LFA-3 shown in SEQ ID NO: 22. Fig. 4 reveals that sheep ΔD2 protein lacks D2 region (underlined portion) which exists in human LFA-3.

### f. Preparation of expression vector for sheep ΔD2 protein in E. coli

In order to make a cDNA coding for sheep ΔD2 protein express in E. coli, an expression vector was constructed.

That is, the inserted DNA contained in the cDNA clone SL-40 obtained in the above-mentioned e, was taken out by cleaving it with restriction enzyme EcoRI, and was subcloned into EcoRI site of plasmid pUC18. Successively such a plasmid was subjected to PCR. The used 5' primer is shown in SEQ ID NO: 23. This primer is comprised of BamHI recognition sequence, NcoI recognition sequence and a DNA sequence deduced from the sequence of the 1st-7th amino acids of SEQ ID NO: 18. The used 3' primer is shown in SEQ ID NO: 24. The primer is comprised of PstI recognition sequence, a sequence of termination codon, and the sequence of the 301st-277th bases and that of the 371th-358th bases of SEQ ID NO: 18. PCR was carried out using the primers of SEQ ID NO: 23 and SEQ ID NO: 24 to amplify a DNA fragment. Thus amplified DNA fragment was cleaved with restriction eyzymes BamHI and PstI, and thus obtained fragment was inserted into BamHI-PstI site of M13mp19 phage vector. A base sequence of the inserted DNA was confirmed by dideoxy method. Then, a gene coding for sheep D1HC protein was taken out from M13 phage vector by cleaving it with restriction enzymes NcoI and PstI. Thus obtained DNA was connected to NcoI-PstI site of vector pKK233-2 (made by Pharmacia) for expression to give an expression vector. Thus obtained expression vector is referred to as pKSLD1HC. SEQ ID NO: 25 shows the base sequence from the initiation codon to the termination codon coding for sheep D1HC protein derivative contained in the pKSLD1HC.

### g. Preparation of expression vector for sheep D1HC protein derivative containing cysteine residue (hereinafter referred to as sheep D1HCcys protein) in E. coli

In order to make a cDNA coding for sheep D1HC protein having cysteine residue at the carboxyl terminus express in E. coli, an expression vector was constructed. The DNA of plasmid pKSLD1HC having the cDNA of sheep D1HC protein obtained in the above-mentioned f was used as a template to carry out PCR. The used 5' primer is the primer used in the above-mentioned f and shown in SEQ ID NO: 23. The used 3' primer is shown in SEQ ID NO: 26. The primer is comprised of HindIII recognition sequence, PstI recognition sequence, a sequence of termination codon, a sequence coding for cysteine residue and then a following sequence of 318th-295th bases of SEQ ID NO: 25. PCR was carried out using the primers of SEQ ID NO: 23 and SEQ ID NO: 26 to amplify a DNA fragment. Thus amplified DNA fragment was cleaved with restriction enzymes BamHI and PstI. The cleaved fragment was inserted into BamHI-PstI site of M13mp19 phage vector to give a recombinant vector. A base sequence of the introduced DNA was confirmed by dideoxy method. Then, a gene coding for sheep D1HCcys protein was taken out from M13 phage vector by cleaving it with restriction enzymes NcoI and PstI. Thus obtained DNA was connected to NcoI-PstI site of vector pKK233-2 (made by Pharmacia) for expression to give expression vector pKSLD1HCcys. SEQ ID NO: 27 shows the base sequence from the initiation codon to the termination codon coding for sheep D1HCcys protein contained in the pKSLD1HCcys.

### h. Production of monomer of sheep D1HCcys protein

E. coli JM 109 (made by TAKARA SHUZO Co.) was precultured which had the expression vector pKSLD1HCcys for sheep D1HCcys protein. Then, the precultured E. coli was inoculated in 100 mℓ of LB medium (0.5 % yeast extract (made by Difco), 1 % bactotryptone (made by Difco), 1 % NaCℓ) containing 10 mg of ampicillin, and shaking culture was carried out at 37°C in a 500 mℓ - Sakaguchi flask until absorbance at 600 nm of the medium containing E. coli became 0.3 by means of Spectrophotometer U-2000 made by Hitachi, Ltd. Successively thereto was added IPTG so as to give a final concentration of 1 mM, and further culture was continued for 6 hours. The cultured cells were collected by centrifugation (1000×g, 10 minutes, 4°C ), and suspended in 10 mℓ of 50 mM Tris-hydrochloric acid (pH 8.0) containing 50 mM EDTA, 5 % Triton X-100 and 8 % sucrose. Thereto was added lysozyme so as to give a final concentration of 0.1 %. The cell suspension was sonicated for 10 minutes by a sonicator (made by BRASON Co.) after cooling it sufficiently on ice. After the above-mentioned procedure were repeated 3 times, the suspension was centrifuged (6000×g, 10 minutes, 4°C ) to give an insoluble precipitate fraction. The precipitate was washed with 50 % glycerol and ethanol to give inclusion bodies of the produced D1HCcys protein.

### ② Solubilization, renaturation and dimerization of inclusion bodies containing sheep D1HCcys protein

Thus obtained inclusion bodies mentioned above (12.5 mg) were dissolved in 80 mℓ of a buffer (pH 8.5) containing 8M urea, 50 mM Tris, 2 mM EDTA and 50 mM 2-mercaptoethanol, stirred overnight at 4°C and centrifuged (6000×g, 10 minutes, 4°C ) to give a supernatant. To 80 mℓ of the supernatant was added 170 mℓ of a solution containing 50 mM Tris, 2 mM EDTA and 50 mM 2-mercaptoethanol to give a final amount of 250 mℓ. The mixture was dialyzed overnight against 10L of a solution containing 50 mM Tris and 2 mM EDTA (pH 8.5). After concentrating the dialyzed liquid with an ultrafiltration membrane of cut-off molecular weight 3000, the concentrate was applied to a gel filtration column (Sephacryl S-200 made by Pharmacia) which was equilibrated with a solution (pH 8.5) containing 50 mM Tris and 2 mM EDTA in the same manner as in Example 1, a dimer fraction of sheep D1HCcys protein was obtained (550 µg as a whole yield). A purified product was obtained by concentrating the eluate.

### TEST EXAMPLE 1

### Immunosuppressive effect of human D1Hcys protein molecule (monomer and dimer) and sheep D1HCcys (monomer and dimer)

With respect to an activation reaction of T cells by PPD (Purified Protein Derivative) antigen, which was used generally as a model system in vitro for an immunological reaction, the immunosuppressive effects of a dimer of human D1Hcys protein and a dimer of sheep D1HCcys protein of the present invention were studied by examining to what degree each dimer suppressed the activation reaction and how each dimer changed expression of a surface antigen of the T cells.

### (Activation reaction by PPD)

Normal human peripheral blood cells, of which concentration was adjusted to 1 × 10⁶/mℓ with RPMI-1640 (made by Sigma) containing 10 % autologous serum and 10⁻⁶ M 2-mercaptoethanol, were poured into a 96 well flat-bottomed plate in 100 µℓ portions. PPD (made by Japan B.C.G Seizo Co., Ltd.) was added to each well so that its final concentration became 1 µg/mℓ, and simultaneously a dimer of human D1Hcys protein and a monomer of human D1Hcys protein were added to each well so that its final concentration became about 5 µg/mℓ and 60 µg/mℓ, respectively. The cells were cultured for five days (in an incubator, 5 % CO₂, 37°C ). Thereafter, 1 µCi of ³H was added to each well, and the cells were cultured for five hours. Then, the cells were collected using a cell-harvester (LABO MASH, made by LABO SCIENCE) to measure radioactivity of ³H which was incorporated into the cells by Scintillation counter LSC-700 (made by Aloka). There were also measured wells to which only the cells were added as a control, wells to which the cells were added with an equivalent amount (10 µℓ) of PBS instead of a protein, wells to which the cells were added with only a dimer of human D1Hcys protein and wells to which the cells were added with only a monomer of human D1Hcys protein. The similar measurement was performed on a sheep D1HCcys protein molecule.

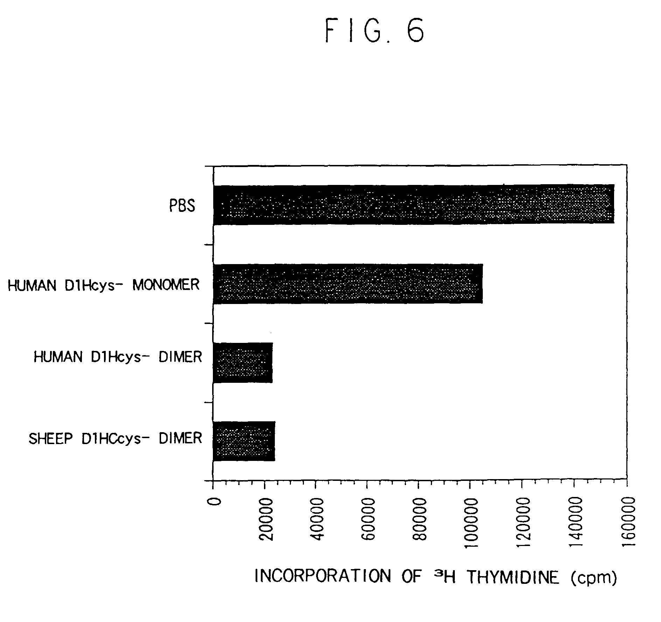

As a result, a dimer of human D1Hcys protein showed an 85 % immunosuppressive activity at a final concentration of about 5 µg/mℓ as shown in Fig. 6, while a monomer thereof showed only about a 30 % immunosuppressive activity even at a final concentration of 60 µg/mℓ. Also a dimer of sheep D1Hcys protein showed a 90 % immunosuppressive activity at a final concentration of about 5 µg/mℓ and a monomer thereof showed a 33 % immunosuppressive activity, the data is not shown in a figure.

### (Flow cytometric analysis of surface antigen)

An influence of a dimer of human D1Hcys protein was examined on expression of CD3 molecule as a surface antigen of human T cells.

Normal human peripheral blood cells, of which concentration was adjusted to 1 × 10⁶/mℓ with RPMI-1640 containing 10 % autologous serum and 10⁻⁵ M 2-mercaptoethanol, were poured into a 96 well flat-bottomed plate in 100 µℓ portions. After adding a dimer of human D1Hcys protein so that a final concentration became 5 µg/mℓ, the cells were incubated for eight days at 37°C in a CO₂ incubator. As a control, the cells added with an equivalent amount of PBS were cultured under the same conditions. After the culture was completed, the cells were collected, and a flow cytometric analysis was performed by EPICS ELITE (made by Coulter) by using an anti-CD3 antibody labeled with a fluorescence (made by Ortho Diagnostics Systems). A similar analysis was performed on a dimer of sheep D1HCcys protein.

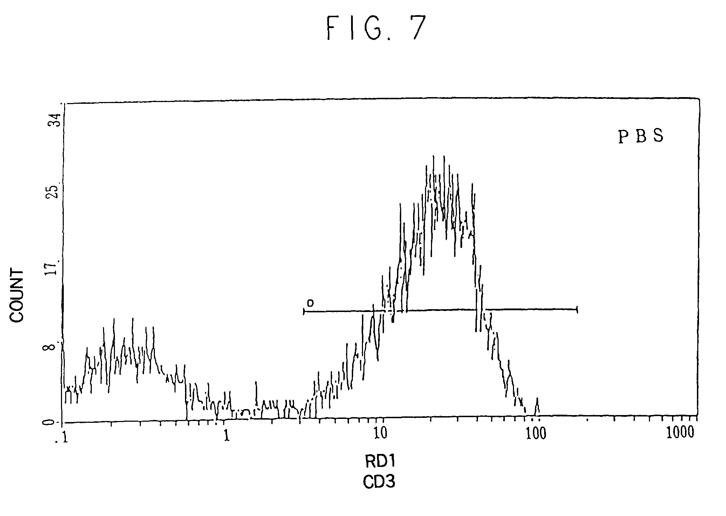

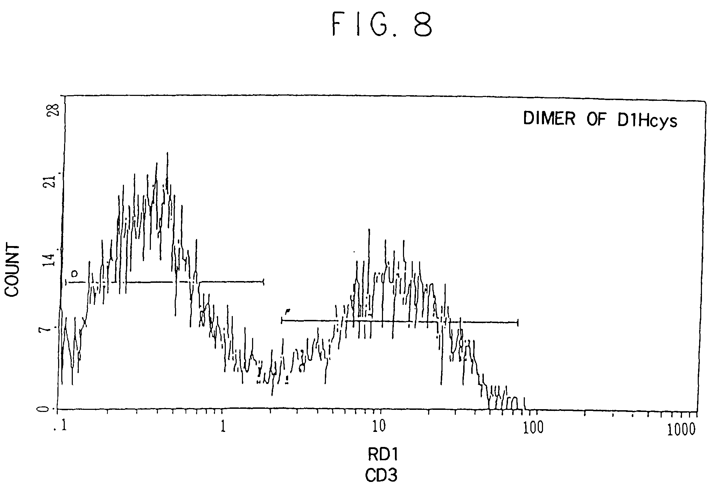

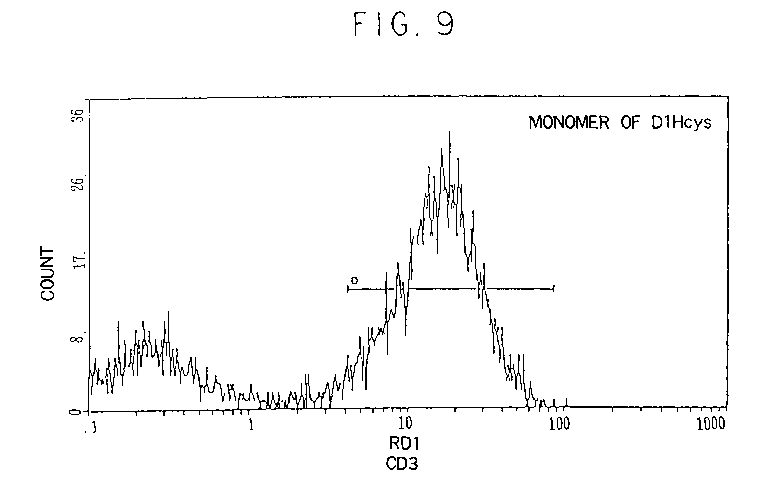

As a result, expression of CD3 molecule on human T cells which were incubated with a dimer of human D1Hcys protein declined significantly (Fig. 8), compared with non-treated group (Fig. 7). Fig. 9 shows a pattern of a flow cytometry when the cells were treated with a monomer of the D1Hcys protein. And a similar result was obtained on a dimer of sheep D1HCcys protein.

The above results strongly suggest that CD3 molecule is indispensable when an antigen such as PPD activates T cells, and that a dimer molecule of human D1Hcys protein and a dimer molecule of sheep D1HCcys protein suppress expression of CD3 molecule to bring about immunosuppression, because the suppression of the expression relates to that of the T cell activation. Accordingly the immunosuppression mechanism by the dimers is clearly different from that by an anti-CD2 antibody. Also, because a dimer of human D1Hcys protein and a dimer of sheep D1HCcys protein do not have Fc domain of an antibody, there occurs no cytotoxic reaction by NK cells and the like, and they show an ideal immunosuppressive action with little side effects.

The following consideration had been made on the immunosuppression mechanism of the dimer molecules of the present invention. That is, autoimmune disease and a rejection reaction in organ transplantation are fundamentally responses of T cells to an autoantigen or a foreign antigen, and originate from contact of T cells with macrophages or dendritic cells, which present antigens thereof. As bonds of molecules on cells relating to the contact reaction, are generally known bonds of CD2-LFA-3 and CD28-B7, which are called as accessory molecules, as well as a major bond of a complex between an antigen on an antigen presenting side, and MHC (Major HistoCompatible antigen) molecule, to TCR (T Cell Receptor) on a T cell side. According to the report by P. Bretscher et al. (see Science, vol.169, p.1042(1970)), the signal caused by the (MHC + an antigen)-TCR bond and another signal caused by another binding pair are indispensable to activate T cells. Based on this consideration, the following two mechanisms can be assumed as an immunosuppressive mechanism of the dimer of the protein according to the present invention.
1) The bond of CD2-LFA-3 is indispensable as the second signal in the above-mentioned two signal theory, and the dimer of the protein according to the present invention inhibits this binding. As a result, since there is only one signal from the (MHC + an antigen)-TCR bond, T cells remain in a non-respondent state.
   This induces an antigen specific unresponsiveness of T cells, and is the most ideal immunosuppression if side effects are taken into a consideration.
2) The bond of the dimer of the protein according to the present invention to CD2 molecule produces any signal which suppresses a function of T cells. This is not specific to an antigen, and the possibility that it acts nonselectively on T cells is high. However, since the dimer of the present invention causes no injury induced by such a molecule as having Fc domain, it may be considered that the dimer has comparatively slight side effects.

It is thought that the dimer of the protein according to the present invention shows an immunosuppressive activity by both or either of the above-mentioned mechanisms.

### EXAMPLE 3

### a. Making of immunosuppressive T cells from fraction in which ratio of T cells in human peripheral blood cells has been made high

To a 25 cm²-culture flask was added 10 mℓ of a medium containing normal human peripheral blood cells, of which concentration was adjusted to 1 × 10⁶/mℓ with RPMI-1640 (made by Sigma) containing 10 % autologous serum. After culturing the cells overnight, the culture medium containing suspended cells was collected, and was transferred to a 12 well-plate in 2 mℓ portions. To the above-mentioned well was added a dimer of human D1Hcys protein or a dimer of sheep D1HCcys protein so that a final concentration thereof became 0.5 µg/mℓ. The cells were cultured for seven days (in an incubator, 5 % CO₂, 37°C ). As a control, a culture system of cells added only with 200 µℓ of PBS was used. Then, the cells were washed three times with serum-free RPMI-1640 culture medium, and were suspended in RPMI-1640 culture medium containing 10 % autologous serum so that a concentration thereof became 3.3 × 10⁶/mℓ. Further, cell suspensions diluted by 2-fold and 4-fold with the same culture medium were made.

### b. Making of immunosuppressive T cells from whole fraction of human peripheral blood cells

To each well of a 12 well-plate were added normal human peripheral blood cells, of which concentration was adjusted to 1 × 10⁶/mℓ with RPMI- 1640 containing 10 % autologous serum (made by Sigma), in 2 mℓ portions. To a part of the above-mentioned wells was added a dimer of human D1Hcys protein or a dimer of sheep D1HCcys protein so that a final concentration became 0.5 µg/mℓ, and the cells were cultured for seven days (in an incubator, 5 % CO₂, 37°C). Simultaneously, to the above-mentioned wells to which the protein was added, and to another wells, were/was further added GM-CSF (made by RD systems) (a final concentration 10 ng/mℓ) and/or IL-2 (made by Vecton · Diskinson) (a final concentration 20 units/mℓ), and the cells were cultured for seven days (in an incubator, 5 % CO₂, 37°C ). Thereafter, the cells were washed three times with serum-free RPMI-1640 culture medium. The cells were suspended in RPMI-1640 culture medium containing 10 % autologous serum so that a concentration thereof became 0.825 × 10⁶/mℓ.

### c. Preparation of immunosuppressive agent according to the present invention

In the same manner as in the above-mentioned a, a dimer of human D1Hcys protein or a dimer of sheep D1HCcys protein was added to cells, and the cells were cultured and recovered by centrifugation. The cells were washed three times with saline, and thus obtained cells were finally suspended in saline so that a final concentration thereof became 10⁶ to 10⁷/mℓ to give an immunosuppressive agent of the present invention.

### TEST EXAMPLE 2

### a. Suppression of activation reaction specific to antigen (PPD) of T cells by immunosuppressive T cells obtained from T cell fraction of human peripheral blood cells

Normal human peripheral blood cells, of which concentration was adjusted to 1 × 10⁶/mℓ with RPMI-1640 (made by Sigma) containing 10 % autologous serum, were poured into a 96 well round-bottomed plate in 100 µℓ portions. After PPD (made by Japan B.C.G. Seizo Co., Ltd.) was added to each well so that a final concentration became 1 µg/mℓ, simultaneously thereto was added 30 µℓ of each of cell suspensions in three kinds of concentrations obtained in Example 1-a, which were treated with a dimer of human D1Hcys protein. The cells were cultured for five days (in an incubator, 5 % CO₂, 37°C ). Then, to each well was added 1 µCi of [ ³H] thymidine, and the cells were cultured for 15 hours. The cells were collected by using a cell-harvester (LABO MASH, made by LABO SCIENCE). Radioactivity of ³H incorporated into the cells was measured by Scintillation counter LSC-700 (made by Aloka). As a control, wells to which was added 30 µℓ of RPMI-1640 culture medium containing 10 % autologous serum, instead of the cells obtained in Example 3, were also measured. The similar measurements were performed on cells treated with a dimer of sheep D1HCcys protein. Each sample was measured with n=4.

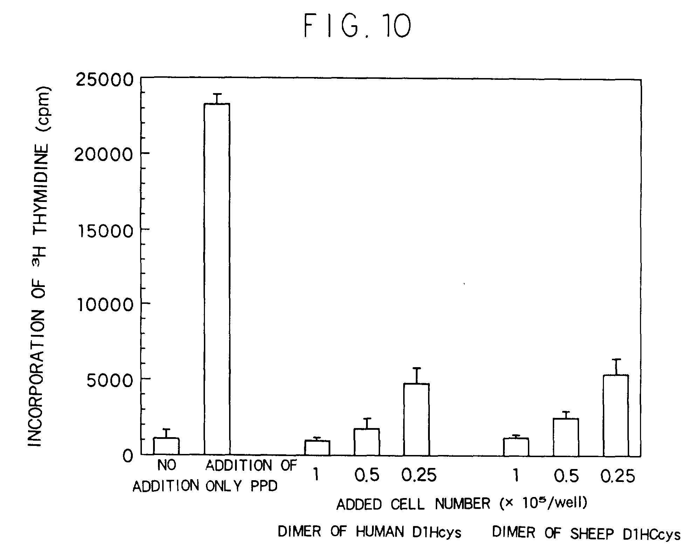

As a result, it was shown that about 80 % suppression was obtained in the case where a quarter amount of cells (which were treated with a dimer of human D1Hcys protein) were added to human cells subjected to a PPD-T cell activating system, and more than 95 % suppression was obtained in cases where a half or equivalent amount of the treated cells were added thereto as shown in Fig. 10. And the same results were obtained on cells treated with a dimer of sheep D1HCcys protein.

### b. Suppression of activation reaction specific to antigen (PPD) of T cells by immunosuppressive T cells obtained by treating whole human peripheral blood cells

Normal human peripheral blood cells, of which concentration was adjusted to 1 × 10⁶/mℓ with RPMI-1640 (made by Sigma) containing 10 % autologous serum, were poured into a 96 well round-bottomed plate in 100 µℓ portions. To each well was added PPD (made by Japan B.C.G. Seizo Co., Ltd.) so that a final concentration became 1 µg/mℓ, and simultaneously thereto was added 30 µℓ of a cell containing-culture medium obtained in Example 3-b wherein the cells were treated with various combinations of a dimer of human D1Hcys protein, GM-CSF and IL-2. The cells were cultured for five days (in an incubator, 5 % CO₂, 37°C). Then, to each well was added 1 µCi of [ ³H] thymidine, and the cells were cultured for 15 hours. The cells were collected by using a cell-harvester (LABO MASH, made by LABO SCIENCE). Radioactivity of ³H incorporated into the cells was measured by Scintillation counter LSC-700 (made by aloka). As a control, wells to which was added 30 µℓ of RPMI-1640 culture medium containing 10 % autologous serum, instead of the cells obtained in Example 3, were also measured. The similar measurements were performed on cells treated by a dimer of sheep D1HCcys protein.

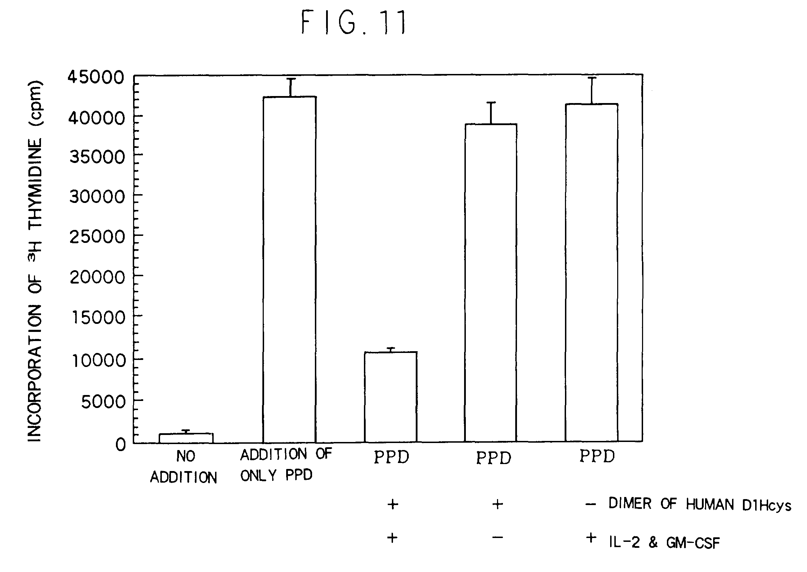

As a result, shown in Fig. 11, in adding human cells which were treated with only a dimer of human D1Hcys, under the coexistence of the above-mentioned dimer, IL-2 and GM-CSF, or under the coexistence of IL-2 and GM-CSF, to the PPD-T cell activating system to culture the cells; in case of adding the cells treated with only a dimer of human D1Hcys protein, almost no suppressive effect could be recognized, while in case of adding simultaneously a dimer of human D1Hcys, GM-CSF and IL-2 to the cells and culturing the cells, the most strong immunosuppressive effect (80 %) could be recognized. And almost no suppressive effect could be recognized in case of adding a combination of GM-CSF and IL-2 to the cells and culturing the cells.

### INDUSTRIAL APPLICABILITY

According to the present invention, there can be provided an immunosuppressive agent having a potent immunosuppressive activity and little side effects.

SEQUENCE LISTING

(1) GENERAL INFORMATION:

(i) TITLE OF INVENTION: CELL ADHESION PROTEIN, IMMUNOSUPPRESSIVE
AGENT CONTAINING THE SAME AND
IMMUNOSUPPRESSIVE AGENT CONTAINING CELL
INDUCED THEREBY

(ii) NUMBER OF SEQUENCES: 28

(2) INFORMATION FOR SEQ ID NO:1:
(i) SEQUENCE CHARACTERISTICS:
(A) LENGTH: 128 amino acids
(B) TYPE: amino acid
(D) TOPOLOGY: linear
(ii) MOLECULE TYPE: protein
(vi) ORIGINAL SOURCE:
(A) ORGANISM: Homo sapiens
(G) CELL TYPE: MOLT-4 cell
(xi) SEQUENCE DESCRIPTION: SEQ ID NO:1:
Met Val Ala Gly Ser Asp Ala Gly Arg Ala Leu Gly Val Leu Ser
1               5                   10                  15

Val Val Cys Leu Leu His Cys Phe Gly Phe Ile Ser Cys Phe Ser
                20                  25                  30
Gln Gln Ile Tyr Gly Val Val Tyr Gly Asn Val Thr Phe His Val
                35                  40                  45
Pro Ser Asn Val Pro Leu Lys Glu Val Leu Trp Lys Lys Gln Lys
                50                  55                  60
Asp Lys Val Ala Glu Leu Glu Asn Ser Glu Phe Arg Ala Phe Ser
                65                  70                  75
Ser Phe Lys Asn Arg Val Tyr Leu Asp Thr Val Ser Gly Ser Leu
                80                  85                  90
Thr Ile Tyr Asn Leu Thr Ser Ser Asp Glu Asp Glu Tyr Glu Met
                95                 100                 105
Glu Ser Pro Asn Ile Thr Asp Thr Met Lys Phe Phe Leu Tyr Val
               110                 115                 120
Leu Gly His Ser Arg His Arg Cys
               125

(2) INFORMATION FOR SEQ ID NO:2:
  (i) SEQUENCE CHARACTERISTICS:
      (A) LENGTH: 133 amino acids
      (B) TYPE: amino acid
      (D) TOPOLOGY: linear
  (ii) MOLECULE TYPE: protein
  (vi) ORIGINAL SOURCE:
      (A) ORGANISM: Homo sapiens
      (G) CELL TYPE: MOLT-4 cell
(xi) SEQUENCE DESCRIPTION: SEQ ID NO:2:
Met Val Ala Gly Ser Asp Ala Gly Arg Ala Leu Gly Val Leu Ser
  1               5                  10                  15
Val Val Cys Leu Leu His Cys Phe Gly Phe Ile Ser Cys Phe Ser
                20                  25                  30
Gln Gln Ile Tyr Gly Val Val Tyr Gly Asn Val Thr Phe His Val
                35                  40                  45
Pro Ser Asn Val Pro Leu Lys Glu Val Leu Trp Lys Lys Gln Lys
                50                  55                  60
Asp Lys Val Ala Glu Leu Glu Asn Ser Glu Phe Arg Ala Phe Ser

                65                  70                  75
Ser Phe Lys Asn Arg Val Tyr Leu Asp Thr Val Ser Gly Ser Leu
70 85 90
Thr Ile Tyr Asn Leu Thr Ser Ser Asp Glu Asp Glu Tyr Glu Met
                95                  100                 105
Glu Ser Pro Asn Ile Thr Asp Thr Met Lys Phe Phe Leu Tyr Val
                110                 115                 120
Leu Gly His Ser Arg His Arg Gly Ser Gly Ser Ser Cys
                125                 130

(2) INFORMATION FOR SEQ ID NO:3:
(i) SEQUENCE CHARACTERISTICS:
(A) LENGTH: 133 amino acids
(B) TYPE: amino acid
(D) TOPOLOGY: linear
(ii) MOLECULE TYPE: protein
(vi) ORIGINAL SOURCE:
(A) ORGANISM: Homo sapiens
(G) CELL TYPE: MOLT-4 cell
(xi) SEQUENCE DESCRIPTION: SEQ ID NO:3:
Met Val Ala Gly Ser Asp Ala Gly Arg Ala Leu Gly Val Leu Ser
1               5                   10                  15
Val Val Cys Leu Leu His Cys Phe Gly Phe Ile Ser Cys Phe Ser
                20                  25                  30
Gln Gln Ile Tyr Gly Val Val Tyr Gly Asn Val Thr Phe His Val
                35                  40                  45
Pro Ser Asn Val Pro Leu Lys Glu Val Leu Trp Lys Lys Gln Lys
                50                  55                  60
Asp Lys Val Ala Glu Leu Glu Asn Ser Glu Phe Arg Ala Phe Ser
                65                  70                  75
Ser Phe Lys Asn Arg Val Tyr Leu Asp Thr Val Ser Gly Ser Leu
                80                  85                  90
Thr Ile Tyr Asn Leu Thr Ser Ser Asp Glu Asp Glu Tyr Glu Met
                95                  100                 105
Glu Ser Pro Asn Ile Thr Asp Thr Met Lys Phe Phe Leu Tyr Val
                110                 115                 120

Leu Gly His Ser Arg His Arg Gly Ser Ser Cys Gly Pro
                125                 130

(2) INFORMATION FOR SEQ ID NO:4:
  (i) SEQUENCE CHARACTERISTICS:
      (A) LENGTH: 134 amino acids
      (B) TYPE: amino acid
      (D) TOPOLOGY: linear
  (ii) MOLECULE TYPE: protein
  (vi) ORIGINAL SOURCE:
      (A) ORGANISM: Ovis
      (G) CELL TYPE: Leukocyte
  (xi) SEQUENCE DESCRIPTION: SEQ ID NO:4:
Met Ala Ala Gly Ser Ala Pro Gly Cys Ala Leu Gly Ala Leu Gly
                5                   10                  15
Leu Val Cys Leu Phe Leu Lys Leu Asp Phe Ile Ser Cys Val Ser
                20                  25                  30
Gln Asp Ile Tyr Gly Ala Met Asn Gly Asn Val Thr Phe Tyr Val
                35                  40                  45
Ser Glu Ser Gln Pro Phe Thr Glu Ile Met Trp Lys Lys Gly Lys
                50                  55                  60
Asp Lys Val Val Glu Trp Asp Gln Thr Ser Gly Leu Glu Ala Phe
                65                  70                  75
Gln Ser Phe Lys Asn Arg Val His Leu Asp Ile Val Ser Gly Asn
                80                  85                  90
Leu Thr Ile Thr Gly Leu Thr Lys Leu Asp Glu Asp Val Tyr Glu
                95                  100                 105
Ile Glu Ser Pro Ser Val Lys Lys Ser Ser Gln Phe His Leu Arg
                110                 115                 120
Val Ile Asp Tyr Ala Arg His Arg Phe Ser Gly Thr Ser Cys
                125                 130

(2) INFORMATION FOR SEQ ID NO:5:
  (i) SEQUENCE CHARACTERISTICS:
      (A) LENGTH: 107 amino acids
      (B) TYPE: amino acid

(D) TOPOLOGY: linear
(ii) MOLECULE TYPE: protein
(vi) ORIGINAL SOURCE:
(A) ORGANISM: Ovis
(G) CELL TYPE: Leukocyte
(ix) FEATURE: The 2nd Val and the 3rd Ser in this sequence are amino
acids obtained from the 2nd codon GTT and the 3rd codon
TCC in the nucleic acid sequence of SEQ ID NO:11,
respectively.
(xi) SEQUENCE DESCRIPTION: SEQ ID NO:5:
Met Val Ser Gln Asp Ile Tyr Gly Ala Met Asn Gly Asn Val Thr
                5                   10                  15
Phe Tyr Val Ser Glu Ser Gln Pro Phe Thr Glu Ile Met Trp Lys
                20                  25                  30
Lys Gly Lys Asp Lys Val Val Glu Trp Asp Gln Thr Ser Gly Leu
                35                  40                  45
Glu Ala Phe Gln Ser Phe Lys Asn Arg Val His Leu Asp Ile Val
                50                  55                  60
Ser Gly Asn Leu Thr Ile Thr Gly Leu Thr Lys Leu Asp Glu Asp
                65                  70                  75
Val Tyr Glu Ile Glu Ser Pro Ser Val Lys Lys Ser Ser Gln Phe
                80                  85                  90
His Leu Arg Val Ile Asp Tyr Ala Arg His Arg Phe Ser Gly Thr
                95                  100                 105
Ser Cys

(2) INFORMATION FOR SEQ ID NO:6:
(i) SEQUENCE CHARACTERISTICS:
(A) LENGTH: 107 amino acids
(b) TYPE: amino acid
(D) TOPOLOGY: linear
(ii) MOLECULE TYPE: protein
(vi) ORIGINAL SOURCE:
(A) ORGANISM: Ovis
(G) CELL TYPE: Leukocyte
(ix) FEATURE: The 2nd Val and the 3rd Ser in this sequence are amino

acids obtained from the 2nd codon GTA and the 3rd
codon AGT in the nucleic acid sequence of SEQ ID NO:
12, respectively.
(xi) SEQUENCE DESCRIPTION: SEQ ID NO:6:
Met Val Ser Gln Asp Ile Tyr Gly Ala Met Asn Gly Asn Val Thr
                5                   10                  15
Phe Tyr Val Ser Glu Ser Gln Pro Phe Thr Glu Ile Met Trp Lys
                20                  25                  30
Lys Gly Lys Asp Lys Val Val Glu Trp Asp Gln Thr Ser Gly Leu
                35                  40                  45
Glu Ala Phe Gln Ser Phe Lys Asn Arg Val His Leu Asp Ile Val
                50                  55                  60
Ser Gly Asn Leu Thr Ile Thr Gly Leu Thr Lys Leu Asp Glu Asp
                65                  70                  75
Val Tyr Glu Ile Glu Ser Pro Ser Val Lys Lys Ser Ser Gln Phe
                80                  85                  90
His Leu Arg Val Ile Asp Tyr Ala Arg His Arg Phe Ser Gly Thr
                95                  100                 105
Ser Cys

(2) INFORMATION FOR SEQ ID NO:7:
(i) SEQUENCE CHARACTERISTICS:
(A) LENGTH: 387 base pairs
(B) TYPE: nucleic acid
(C) STRANDEDNESS: single
(D) TOPOLOGY: linear
(ii) MOLECULE TYPE: other nucleic acid
(A) DESCRIPTION: synthetic DNA
(vi) ORIGINAL SOURCE:
(A) ORGANISM: Homo sapiens
(G) CELL TYPE: MOLT-4 cell
(xi) SEQUENCE DESCRIPTION: SEQ ID NO:7:
ATGGTTGCTG GGAGCGACGC GGGGCGGGCC CTGGGGGTCC TCAGCGTGGT CTGCCTGCTG 60
CACTGCTTTG GTTTCATCAG CTGTTTTTCC CAACAAATAT ATGGTGTTGT GTATGGGAAT 120
GTAACTTTCC ATGTACCAAG CAATGTGCCT TTAAAAGAGG TCCTATGGAA AAAACAAAAG 180
GATAAAGTTG CAGAACTGGA AAATTCTGAA TTCAGAGCTT TCTCATCTTT TAAAAATAGG 240

GTTTATTTAG ACACTGTGTC AGGTAGCCTC ACTATCTACA ACTTAACATC ATCAGATGAA 300
GATGAGTATG AAATGGAATC GCCAAATATT ACTGATACCA TGAAGTTCTT TCTTTATGTG 360
CTTGGTCATT CAAGACACAG ATGTTGA 387

(2) INFORMATION FOR SEQ ID NO:8:
(i) SEQUENCE CHARACTERISTICS:
(A) LENGTH: 402 base pairs
(B) TYPE: nucleic acid
(C) STRANDEDNESS: single
(D) TOPOLOGY: linear
(ii) MOLECULE TYPE: other nucleic acid
(A) DESCRIPTION: synthetic DNA
(vi) ORIGINAL SOURCE:
(A) ORGANISM: Homo sapiens
(G) CELL TYPE: MOLT-4 cell
(xi) SEQUENCE DESCRIPTION: SEQ ID NO:8:
ATGGTTGCTG GGAGCGACGC GGGGCGGGCC CTGGGGGTCC TCAGCGTGGT CTGCCTGCTG 60
CACTGCTTTG GTTTCATCAG CTGTTTTTCC CAACAAATAT ATGGTGTTGT GTATGGGAAT 120
GTAACTTTCC ATGTACCAAG CAATGTGCCT TTAAAAGAGG TCCTATGGAA AAAACAAAAG 180
GATAAAGTTG CAGAACTGGA AAATTCTGAA TTCAGAGCTT TCTCATCTTT TAAAAATAGG 240
GTTTATTTAG ACACTGTGTC AGGTAGCCTC ACTATCTACA ACTTAACATC ATCAGATGAA 300
GATGAGTATG AAATGGAATC GCCAAATATT ACTGATACCA TGAAGTTCTT TCTTTATGTG 360
CTTGGTCATT CAAGACACAG AGGAAGTGGA AGTAGTTGTT GA 402

(2) INFORMATION FOR SEQ ID NO:9:
(i) SEQUENCE CHARACTERISTICS:
(A) LENGTH: 402 base pairs
(B) TYPE: nucleic acid
(C) STRANDEDNESS: single
(D) TOPOLOGY: linear
(ii) MOLECULE TYPE: other nucleic acid
(A) DESCRIPTION: synthetic DNA
(vi) ORIGINAL SOURCE:
(A) ORGANISM: Homo sapiens
(G) CELL TYPE: MOLT-4 cell
(xi) SEQUENCE DESCRIPTION: SEQ ID NO:9:

ATGGTTGCTG GGAGCGACGC GGGGCGGGCC CTGGGGGTCC TCAGCGTGGT CTGCCTGCTG 60
CACTGCTTTG GTTTCATCAG CTGTTTTTCC CAACAAATAT ATGGTGTTGT GTATGGGAAT 120
GTAACTTTCC ATGTACCAAG CAATGTGCCT TTAAAAGAGG TCCTATGGAA AAAACAAAAG 180
GATAAAGTTG CAGAACTGGA AAATTCTGAA TTCAGAGCTT TCTCATCTTT TAAAAATAGG 240
GTTTATTTAG ACACTGTGTC AGGTAGCCTC ACTATCTACA ACTTAACATC ATCAGATGAA 300
GATGAGTATG AAATGGAATC GCCAAATATT ACTGATACCA TGAAGTTCTT TCTTTATGTG 360
CTTGGTCATT CAAGACACAG AGGAAGTAGT TGTGGCCCAT GA 402

(2) INFORMATION FOR SEQ ID NO:10:
(i) SEQUENCE CHARACTERISTICS:
(A) LENGTH: 405 base pairs
(B) TYPE: nucleic acid
(C) STRANDEDNESS: single
(D) TOPOLOGY: linear
(ii) MOLECULE TYPE: other nucleic acid
(A) DESCRIPTION: synthetic DNA
(vi) ORIGINAL SOURCE:
(A) ORGANISM: Ovis
(G) CELL TYPE: Leukocyte
(xi) SEQUENCE DESCRIPTION: SEQ ID NO:10:
ATGGCCGCAG GGAGTGCCCC TGGGTGTGCC CTGGGGGCCC TTGGACTGGT CTGCCTGTTC 60
CTCAAATTGG ATTTCATCAG CTGTGTTTCC CAAGATATTT ATGGAGCTAT GAATGGGAAT 120
GTAACCTTTT ACGTTTCAGA GTCTCAACCG TTTACAGAGA TTATGTGGAA GAAGGGGAAG 180
GATAAAGTTG TAGAATGGGA TCAAACATCT GGACTCGAAG CTTTTCAGTC TTTTAAAAAT 240
AGAGTTCATT TAGACATTGT GTCAGGTAAC CTCACCATCA CCGGGTTAAC AAAATTAGAT 300
GAAGATGTGT ATGAAATTGA ATCCCCAAGT GTTAAAAAGA GCTCCCAGTT CCACCTCAGA 360
GTGATTGATT ATGCAAGGCA TAGGTTTTCT GGGACGTCGT GTTAG 405

(2) INFORMATION FOR SEQ ID NO:11:
(i) SEQUENCE CHARACTERISTICS:
(A) LENGTH: 324 base pairs
(B) TYPE: nucleic acid
(C) STRANDEDNESS: single
(D) TOPOLOGY: linear
(ii) MOLECULE TYPE: other nucleic acid
(A) DESCRIPTION: synthetic DNA

(vi) ORIGINAL SOURCE:
(A) ORGANISM: Ovis
(G) CELL TYPE: Leukocyte
(xi) SEQUENCE DESCRIPTION: SEQ ID NO:11:
ATGGTTTCCC AAGATATTTA TGGAGCTATG AATGGGAATG TAACCTTTTA CGTTTCAGAG 60
TCTCAACCGT TTACAGAGAT TATGTGGAAG AAGGGGAAGG ATAAAGTTGT AGAATGGGAT 120
CAAACATCTG GACTCGAAGC TTTTCAGTCT TTTAAAAATA GAGTTCATTT AGACATTGTG 180
TCAGGTAACC TCACCATCAC CGGGTTAACA AAATTAGATG AAGATGTGTA TGAAATTGAA 240
TCCCCAAGTG TTAAAAAGAG CTCCCAGTTC CACCTCAGAG TGATTGATTA TGCAAGGCAT 300
AGGTTTTCTG GGACGTCGTG TTAG 324
(2) INFORMATION FOR SEQ ID NO:12:
(i) SEQUENCE CHARACTERISTICS:
(A) LENGTH: 324 base pairs
(B) TYPE: nucleic acid
(C) STRANDEDNESS: single
(D) TOPOLOGY: linear
(ii) MOLECULE TYPE: other nucleic acid
(A) DESCRIPTION: synthetic DNA
(vi) ORIGINAL SOURCE:
(A) ORGANISM: Ovis
(G) CELL TYPE: Leukocyte
(xi) SEQUENCE DESCRIPTION: SEQ ID NO:12:
ATGGTAAGTC AAGATATTTA TGGAGCTATG AATGGGAATG TAACCTTTTA CGTTTCAGAG 60
TCTCAACCGT TTACAGAGAT TATGTGGAAG AAGGGGAAGG ATAAAGTTGT AGAATGGGAT 120
CAAACATCTG GACTCGAAGC TTTTCAGTCT TTTAAAAATA GAGTTCATTT AGACATTGTG 180
TCAGGTAACC TCACCATCAC CGGGTTAACA AAATTAGATG AAGATGTGTA TGAAATTGAA 240
TCCCCAAGTG TTAAAAAGAG CTCCCAGTTC CACCTCAGAG TGATTGATTA TGCAAGGCAT 300
AGGTTTTCTG GGACGTCGTG TTAG 324
(2) INFORMATION FOR SEQ ID NO:13:
(i) SEQUENCE CHARACTERISTICS:
(A) LENGTH: 42 base pairs
(B) TYPE: nucleic acid
(C) STRANDEDNESS: single
(D) TOPOLOGY: linear

(ii) MOLECULE TYPE: other nucleic acid
(A) DESCRIPTION: synthetic DNA
(xi) SEQUENCE DESCRIPTION: SEQ ID NO:13:
TTTTCTGCAG AAGCTTCCAT GGTTGCTGGG AGCGACGCGG GG 42

(2) INFORMATION FOR SEQ ID NO:14:
(i) SEQUENCE CHARACTERISTICS:
(A) LENGTH: 47 base pairs
(B) TYPE: nucleic acid
(C) STRANDEDNESS: single
(ii) MOLECULE TYPE: other nucleic acid
(A) DESCRIPTION: synthetic DNA
(xi) SEQUENCE DESCRIPTION: SEQ ID NO:14:
TTTTGTCGAC AGATCTATCA ACATCTGTGT CTTGAATGAC CAAGCAC 47

(2) INFORMATION FOR SEQ ID NO:15:
(i) SEQUENCE CHARACTERISTICS:
(A) LENGTH: 29 amino acids
(B) TYPE: amino acid
(D) TOPOLOGY: unknown
(ii) MOLECULE TYPE: peptide
(iii) HYPOTHETICAL: YES
(v) FRAGMENT TYPE: N-terminal peptide
(ix) FEATURE: The 12th and the 28th Xaa in this sequence represents
one of the natural amino acids, the 12th one is
preferably Ser. The 1st amino acid is either Val or
Phe, and the 3rd one is either Gln or Ser.
(xi) SEQUENCE DESCRIPTION: SEQ ID NO:15:
Val/Phe Ser Gln/Ser Asp Ile Tyr Gly Ala Met Asn Gly Xaa Val Thr Phe Tyr
1 5 10 15
Val Ser Glu Ser Gln Pro Phe Thr Glu Ile Met Xaa Lys
20 25

(2) INFORMATION FOR SEQ ID NO:16:
(i) SEQUENCE CHARACTERISTICS:
(A) LENGTH: 29 base pairs

(B) TYPE: nucleic acid
(C) STRANDEDNESS: single
(D) TOPOLOGY: linear
(ii) MOLECULE TYPE: other nucleic acid
(A) DESCRIPTION: synthetic DNA
(iii) HYPOTHETICAL: NO
(xi) SEQUENCE DESCRIPTION: SEQ ID NO:16:
GTTGGATCCT TYWSNCARGA YATHTAYGG 29

(2) INFORMATION FOR SEQ ID NO:17:
(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: other nucleic acid
(A) DESCRIPTION: synthetic DNA
(iii) HYPOTHETICAL: NO
(iv) ANTI-SENSE: YES
(xi) SEQUENCE DESCRIPTION: SEQ ID NO:17:
ACACTGCAGC ATDATYTCNG TRAANGG 27

(2) INFORMATION FOR SEQ ID NO:18:
(i) SEQUENCE CHARACTERISTICS:
(A) LENGTH: 393 base pairs
(B) TYPE: nucleic acid
(C) STRANDEDNESS: single
(D) TOPOLOGY: linear
(ii) MOLECULE TYPE: cDNA to mRNA
(iii) HYPOTHETICAL: NO
(vi) ORIGINAL SOURCE:
(A) ORGANISM: Ovis
(G) CELL TYPE: Leukocyte
(xi) SEQUENCE DESCRIPTION: SEQ ID NO:18:
GTT TCC CAA GAT ATT TAT GGA GCT ATG AAT GGG AAT GTA ACC TTT TAC 48
Val Ser Gln Asp Ile Tyr Gly Ala Met Asn Gly Asn Val Thr Phe Tyr

1                   5                   10                  15
GTT TCA GAG TCT CAA CCG TTT ACA GAG ATT ATG TGG AAG AAG GGG AAG      96
Val Ser Glu Ser Gln Pro Phe Thr Glu Ile Met Trp Lys Lys Gly Lys
            20                  25                  30
GAT AAA GTT GTA GAA TGG GAT CAA ACA TCT GGA CTC GAA GCT TTT CAG     144
Asp Lys Val Val Glu Trp Asp Gln Thr Ser Gly Leu Glu Ala Phe Gln
        35                  40                  45
TCT TTT AAA AAT AGA GTT CAT TTA GAC ATT GTG TCA GGT AAC CTC ACC     192
Ser Phe Lys Asn Arg Val His Leu Asp Ile Val Ser Gly Asn Leu Thr
    50                  55                  60
ATC ACC GGG TTA ACA AAA TTA GAT GAA GAT GTG TAT GAA ATT GAA TCC     240
Ile Thr Gly Leu Thr Lys Leu Asp Glu Asp Val Tyr Glu Ile Glu Ser
65                  70                  75                  80
CCA AGT GTT AAA AAG AGC TCC CAG TTC CAC CTC AGA GTG ATT GAT TAT     288
Pro Ser Val Lys Lys Ser Ser Gln Phe His Leu Arg Val Ile Asp Tyr
                85                  90                  95
GCA AGG CAT AGG TAT GTG CTT TTT GCC ATA CTG CCA GCA GTA ATA TGT     336
Ala Arg His Arg Tyr Val Leu Phe Ala Ile Leu Pro Ala Val Ile Cys
            100                 105                 110
GGC TTG CTG TTT TTA AAA TGT TTT CTG GGA CGT CGT AGC CAA CGA AAC     384
Gly Leu Leu Phe Leu Lys Cys Phe Leu Gly Arg Arg Ser Gln Arg Asn
        115                 120                 125
TCA GGG CCC                                                         393
Ser Gly Pro
    130

(2) INFORMATION FOR SEQ ID NO:19:
  (i) SEQUENCE CHARACTERISTICS:
      (A) LENGTH: 131 amino acids
      (B) TYPE: amino acid
  (vi) ORIGINAL SOURCE:
      (A) ORGANISM: Ovis
      (G) CELL TYPE: Leukocyte
  (xi) SEQUENCE DESCRIPTION: SEQ ID NO:19:
Val Ser Gln Asp Ile Tyr Gly Ala Met Asn Gly Asn Val Thr Phe Tyr
1               5                   10                  15

Val Ser Glu Ser Gln Pro Phe Thr Glu Ile Met Trp Lys Lys Gly Lys
            20                  25                  30
Asp Lys Val Val Glu Trp Asp Gln Thr Ser Gly Leu Glu Ala Phe Gln
        35                  40                  45
Ser Phe Lys Asn Arg Val His Leu Asp Ile Val Ser Gly Asn Leu Thr
    50                  55                  60
Ile Thr Gly Leu Thr Lys Leu Asp Glu Asp Val Tyr Glu Ile Glu Ser
65                  70                  75                  80
Pro Ser Val Lys Lys Ser Ser Gln Phe His Leu Arg Val Ile Asp Tyr
                85                  90                  95
Ala Arg His Arg Tyr Val Leu Phe Ala Ile Leu Pro Ala Val Ile Cys
            100                 105                 110
Gly Leu Leu Phe Leu Lys Cys Phe Leu Gly Arg Arg Ser Gln Arg Asn
        115                 120                 125
Ser Gly Pro
    130

(2) INFORMATION FOR SEQ ID NO:20:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: other nucleic acid
(A) DESCRIPTION: synthetic DNA
(iii) HYPOTHETICAL: NO
(xi) SEQUENCE DESCRIPTION: SEQ ID NO:20:
AGCTATGAAC GGGAATGTAA CCTT 24

(2) INFORMATION FOR SEQ ID NO:21:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: other nucleic acid

(A) DESCRIPTION: synthetic DNA
(iii) HYPOTHETICAL: NO
(xi) SEQUENCE DESCRIPTION: SEQ ID NO:21:
ACCTTTTACG TTTCAGAGTC TCAA 24

(2) INFORMATION FOR SEQ NO:22:
(i) SEQUENCE CHARACTERISTICS:
(A) LENGTH: 222 amino acids
(B) TYPE: amino acid
(iii) HYPOTHETICAL: NO
(vi) ORIGINAL SOURCE:
(A) ORGANISM: Homo sapiens
(G) CELL TYPE: PBL (Peripheral Blood Lymphocytes)
(xi) SEQUENCE DESCRIPTION: SEQ ID NO:22:
Phe Ser Gln Gln Ile Tyr Gly Val Val Tyr Gly Asn Val Thr Phe His
1               5                   10                  15
Val Pro Ser Asn Val Pro Leu Lys Glu Val Leu Trp Lys Lys Gln Lys
            20                  25                  30
Asp Lys Val Ala Glu Leu Glu Asn Ser Glu Phe Arg Ala Phe Ser Ser
        35                  40                  45
Phe Lys Asn Arg Val Tyr Leu Asp Thr Val Ser Gly Ser Leu Thr Ile
    50                  55                  60
Tyr Asn Leu Thr Ser Ser Asp Glu Asp Glu Tyr Glu Met Glu Ser Pro
65                  70                  75                  80
Asn Ile Thr Asp Thr Met Lys Phe Phe Leu Tyr Val Leu Glu Ser Leu
                85                  90                  95
Pro Ser Pro Thr Leu Thr Cys Ala Leu Thr Asn Gly Ser Ile Glu Val
            100                 105                 110
Gln Cys Met Ile Pro Glu His Tyr Asn Ser His Arg Gly Leu Ile Met
        115                 120                 125
Tyr Ser Trp Asp Cys Pro Met Glu Gln Cys Lys Arg Asn Ser Thr Ser
    130                 135                 140
Ile Tyr Phe Lys Met Glu Asn Asp Leu Pro Gln Lys Ile Gln Cys Thr
145                 150                 155                 160
Leu Ser Asn Pro Leu Phe Asn Thr Thr Ser Ser Ile Ile Leu Thr Thr
                165                 170                 175

Cys Ile Pro Ser Ser Gly His Ser Arg His Arg Tyr Ala Leu Ile Pro
            180                 185                 190
Ile Pro Leu Ala Val Ile Thr Thr Cys Ile Val Leu Tyr Met Asn Gly
        195                 200                 205
Ile Leu Lys Cys Asp Arg Lys Pro Asp Arg Thr Asn Ser Asn
    210                 215                 220

(2) INFORMATION FOR SEQ ID NO:23:
  (i) SEQUENCE CHARACTERISTICS:
      (A) LENGTH: 32 base pairs
      (B) TYPE: nucleic acid
      (C) STRANDEDNESS: single
      (D) TOPOLOGY: linear
  (ii) MOLECULE TYPE: other nucleic acid
      (A) DESCRIPTION: synthetic DNA
  (iii) HYPOTHETICAL: NO
  (xi) SEQUENCE DESCRIPTION: SEQ ID NO:23:
TGGGGATCCA TGGTAAGTCA AGATATTTAT GG                                32

(2) INFORMATION FOR SEQ ID NO:24:
  (i) SEQUENCE CHARACTERISTICS:
      (A) LENGTH: 51 base pairs
      (B) TYPE: nucleic acid
      (C) STRANDEDNESS: single
      (D) TOPOLOGY: linear
  (ii) MOLECULE TYPE: other nucleic acid
      (A) DESCRIPTION: synthetic DNA
  (iii) HYPOTHETICAL: NO
  (iv) ANTI-SENSE: YES
  (xi) SEQUENCE DESCRIPTION: SEQ ID NO:24:
CAACTGCAGC TACGACGTCC CAGAAAACCT ATGCCTTGCA TAATCAATCA C           51

(2) INFORMATION FOR SEQ ID NO:25:
  (i) SEQUENCE CHARACTERISTICS:
      (A) LENGTH: 321 base pairs
      (B) TYPE: nucleic acid

(C) STRANDEDNESS: single
(D) TOPOLOGY: linear
(iii) HYPOTHETICAL: NO
(xi) SEQUENCE DESCRIPTION: SEQ ID NO:25:
ATG GTA AGT CAA GAT ATT TAT GGA GCT ATG AAT GGG AAT GTA ACC TTT 48
Met Val Ser Gln Asp Ile Tyr Gly Ala Met Asn Gly Asn Val Thr Phe
1               5                   10                  15
TAC GTT TCA GAG TCT CAA CCG TTT ACA GAG ATT ATG TGG AAG AAG GGG 96
Tyr Val Ser Glu Ser Gln Pro Phe Thr Glu Ile Met Trp Lys Lys Gly
            20                  25                  30
AAG GAT AAA GTT GTA GAA TGG GAT CAA ACA TCT GGA CTC GAA GCT TTT 144
Lys Asp Lys Val Val Glu Trp Asp Gln Thr Ser Gly Leu Glu Ala Phe
        35                  40                  45
CAG TCT TTT AAA AAT AGA GTT CAT TTA GAC ATT GTG TCA GGT AAC CTC 192
Gln Ser Phe Lys Asn Arg Val His Leu Asp Ile Val Ser Gly Asn Leu
    50                  55                  60
ACC ATC ACC GGG TTA ACA AAA TTA GAT GAA GAT GTG TAT GAA ATT GAA 240
Thr Ile Thr Gly Leu Thr Lys Leu Asp Glu Asp Val Tyr Glu Ile Glu
65                  70                  75                  80
TCC CCA AGT GTT AAA AAG AGC TCC CAG TTC CAC CTC AGA GTG ATT GAT 288
Ser Pro Ser Val Lys Lys Ser Ser Gln Phe His Leu Arg Val Ile Asp
                85                  90                  95
TAT GCA AGG CAT AGG TTT TCT GGG ACG TCG TAG 321
Tyr Ala Arg His Arg Phe Ser Gly Thr Ser
            100                 105

(2) INFORMATION FOR SEQ ID NO:26:
(i) SEQUENCE CHARACTERISTICS:
(A) LENGTH: 46 base pairs
(B) TYPE: nucleic acid
(C) STRANDEDNESS: single
(D) TOPOLOGY: linear
(ii) MOLECULE TYPE: other nucleic acid
(A) DESCRIPTION: synthetic DNA
(iii) HYPOTHETICAL: NO
(iv) ANTI-SENSE: YES

(xi) SEQUENCE DESCRIPTION: SEQ ID NO:26:
TTTTTTCGAA CTGCAGCTAA CACGACGTCC CAGAAAACCT ATGCCT 46

(2) INFORMATION FOR SEQ ID NO:27:
(i) SEQUENCE CHARACTERISTICS:
(A) LENGTH: 324 base pairs
(B) TYPE: nucleic acid
(C) STRANDEDNESS: both
(D) TOPOLOGY: linear
(iii) HYPOTHETICAL: NO
(xi) SEQUENCE DESCRIPTION: SEQ ID NO:27:
ATG GTA AGT CAA GAT ATT TAT GGA GCT ATG AAT GGG AAT GTA ACC TTT 48
Met Val Ser Gln Asp Ile Tyr Gly Ala Met Asn Gly Asn Val Thr Phe
1               5                   10                  15
TAC GTT TCA GAG TCT CAA CCG TTT ACA GAG ATT ATG TGG AAG AAG GGG 96
Tyr Val Ser Glu Ser Gln Pro Phe Thr Glu Ile Met Trp Lys Lys Gly
            20                  25                  30
AAG GAT AAA GTT GTA GAA TGG GAT CAA ACA TCT GGA CTC GAA GCT TTT 144
Lys Asp Lys Val Val Glu Trp Asp Gln Thr Ser Gly Leu Glu Ala Phe
        35                  40                  45
CAG TCT TTT AAA AAT AGA GTT CAT TTA GAC ATT GTG TCA GGT AAC CTC 192
Gln Ser Phe Lys Asn Arg Val His Leu Asp Ile Val Ser Gly Asn Leu
    50                  55                  60
ACC ATC ACC GGG TTA ACA AAA TTA GAT GAA GAT GTG TAT GAA ATT GAA 240
Thr Ile Thr Gly Leu Thr Lys Leu Asp Glu Asp Val Tyr Glu Ile Glu
65                  70                  75                  80
TCC CCA AGT GTT AAA AAG AGC TCC CAG TTC CAC CTC AGA GTG ATT GAT 288
Ser Pro Ser Val Lys Lys Ser Ser Gln Phe His Leu Arg Val Ile Asp
                85                  90                  95
TAT GCA AGG CAT AGG TTT TCT GGG ACG TCG TGT TAG 324
Tyr Ala Arg His Arg Phe Ser Gly Thr Ser Cys
            100                 105

(2) INFORMATION FOR SEQ ID NO:28:
(i) SEQUENCE CHARACTERISTICS:
(A) LENGTH: 753 base pairs

(B) TYPE: nucleic acid
(C) STRANDEDNESS: both
(ii) MOLECULE TYPE: cDNA to mRNA
(iii) HYPOTHETICAL: NO
(vi) ORIGINAL SOURCE:
(A) ORGANISM: Homo sapiens
(xi) SEQUENCE DESCRIPTION: SEQ ID NO:28:
ATG GTT GCT GGG AGC GAC GCG GGG CGG GCC CTG GGG GTC CTC AGC GTG 48
Met Val Ala Gly Ser Asp Ala Gly Arg Ala Leu Gly Val Leu Ser Val
-28 -25 -20 -15
GTC TGC CTG CTG CAC TGC TTT GGT TTC ATC AGC TGT TTT TCC CAA CAA 96
Val Cys Leu Leu His Cys Phe Gly Phe Ile Ser Cys Phe Ser Gln Gln
-10 -5 1
ATA TAT GGT GTT GTG TAT GGG AAT GTA ACT TTC CAT GTA CCA AGC AAT 144
Ile Tyr Gly Val Val Tyr Gly Asn Val Thr Phe His Val Pro Ser Asn
5 10 15 20
GTG CCT TTA AAA GAG GTC CTA TGG AAA AAA CAA AAG GAT AAA GTT GCA 192
Val Pro Leu Lys Glu Val Leu Trp Lys Lys Gln Lys Asp Lys Val Ala
25 30 35
GAA CTG GAA AAT TCT GAA TTC AGA GCT TTC TCA TCT TTT AAA AAT AGG 240
Glu Leu Glu Asn Ser Glu Phe Arg Ala Phe Ser Ser Phe Lys Asn Arg
40 45 50
GTT TAT TTA GAC ACT GTG TCA GGT AGC CTC ACT ATC TAC AAC TTA
ACA 288
Val Tyr Leu Asp Thr Val Ser Gly Ser Leu Thr Ile Tyr Asn Leu Thr
55 60 65
TCA TCA GAT GAA GAT GAG TAT GAA ATG GAA TCG CCA AAT ATT ACT GAT 336
Ser Ser Asp Glu Asp Glu Tyr Glu Met Glu Ser Pro Asn Ile Thr Asp
70 75 80
ACC ATG AAG TTC TTT CTT TAT GTG CTT GAG TCT CTT CCA TCT CCC ACA 384
Thr Met Lys Phe Phe Leu Tyr Val Leu Glu Ser Leu Pro Ser Pro Thr
85 90 95 100
CTA ACT TGT GCA TTG ACT AAT GGA AGC ATT GAA GTC CAA TGC ATG ATA 432
Leu Thr Cys Ala Leu Thr Asn Gly Ser Ile Glu Val Gln Cys Met Ile
105 110 115
CCA GAG CAT TAC AAC AGC CAT CGA GGA CTT ATA ATG TAC TCA TGG GAT 480
Pro Glu His Tyr Asn Ser His Arg Gly Leu Ile Met Tyr Ser Trp Asp

120 125 130
TGT CCT ATG GAG CAA TGT AAA CGT AAC TCA ACC AGT ATA TAT TTT AAG 528
Cys Pro Met Glu Gln Cys Lys Arg Ans Ser Thr Ser Ile Tyr Phe Lys
135 140 145
ATG GAA AAT GAT CTT CCA CAA AAA ATA CAG TGT ACT CTT AGC AAT CCA 576
Met Glu Asn Asp Leu Pro Gln Lys Ile Gln Cys Thr Leu Ser Asn Pro
150 155 160
TTA TTT AAT ACA ACA TCA TCA ATC ATT TTG ACA ACC TGT ATC CCA AGC 624
Leu Phe Asn Thr Thr Ser Ser Ile Ile Leu Thr Thr Cys Ile Pro Ser
165 170 175 180
AGC GGT CAT TCA AGA CAC AGA TAT GCA CTT ATA CCC ATA CCA TTA GCA 672
Ser Gly His Ser Arg His Arg Tyr Ala Leu Ile Pro Ile Pro Leu Ala
185 190 195
GTA ATT ACA ACA TGT ATT GTG CTG TAT ATG AAT GGT ATT CTG AAA TGT 720
Val Ile Thr Thr Cys Ile Val Leu Tyr Met Asn Gly Ile Leu Lys Cys
200 205 210
GAC AGA AAA CCA GAC AGA ACC AAC TCC AAT TGA 753
Asp Arg Lys Pro Asp Arg Thr Asn Ser Asn
215 220

## Claims

1. A dimer of a protein having an amino acid sequence deficient in the 1st to the 28th amino acids in the amino acid sequence of SEQ ID NO: 1 or an analogue thereof, wherein two of said proteins or analogues are connected each other through SH group of cysteine at C-terminus thereof or its vicinity.

2. The dimer of Claim 1, said analogue is one into which, from N-terminus, glycine-serine-glycine-serine-serine is inserted just before cysteine at the C-terminus or its vicinity; or
one into which, from N-terminus, glycine-serine-serine is inserted just before cysteine at the C-terminus or its vicinity, and, from N-terminus, glycine-proline is inserted just after said cysteine.

3. A DNA coding for a protein which is a soluble D2 region-deficient type human LFA-3 deficient in C region containing one cysteine residue added at C-terminus of said LFA-3 or its vicinity, shown in SEQ ID NO: 7.

4. A method for preparing a dimer of a protein, which comprises spontaneously oxidizing a protein having an amino acid sequence deficient in the 1st to the 28th amino acids in the amino acid sequence of SEQ ID NO: 1 or an analogue thereof.

5. An immunosuppressive agent which comprises a dimer of a protein having an amino acid sequence deficient in the 1st to the 28th amino acids in the amino acid sequence of SEQ ID NO: 1 or an analogue thereof, wherein two of said proteins or analogues are connected each other through SH group of cysteine at C-terminus thereof or its vicinity.

6. A dimer of a protein having an amino acid sequence deficient in the 1st to the 28th amino acids in the amino acid sequence of SEQ ID NO: 4, wherein two of said proteins are connected each other through SH group of cysteine at C-terminus thereof or its vicinity.

7. A method for preparing a dimer of protein, which comprises spontaneously oxidizing a protein having an amino acid sequence deficient in the 1st to the 28th amino acids in the amino acid sequence of SEQ ID NO: 4.

8. An immunosuppressive agent which comprises a dimer of a protein having an amino acid sequence deficient in the 1st to the 28th amino acids in the amino acid sequence of SEQ ID NO: 4, wherein two of said proteins are connected each other through SH group of cysteine at C-terminus thereof its vicinity.

9. A dimer of a protein having the amino acid sequence of SEQ ID NO: 5 or 6, wherein two of said proteins are connected each other through SH group of cysteine at C-terminus thereof or its vicinity.

10. A method for preparing a dimer of a protein, which comprises spontaneously oxidizing a protein having the amino acid sequence of SEQ ID NO: 5 or 6.

11. An immunosuppressive agent which comprises a dimer of a protein having the amino acid sequence of SEQ ID NO: 5 or 6, wherein two of said proteins are connected each other through SH group of cysteine at C-terminus thereof or its vicinity.

12. An immunosuppressive agent which comprises as an effective ingredient an immunosuppressive cell which is obtained by culturing a human cell in the presence of a cell adhesion protein.

13. The immunosuppressive agent of Claim 12, wherein said cell adhesion protein is a dimer of a protein having the amino acid sequence of SEQ ID NO: 1 or derivative thereof, wherein two of said proteins or derivatives are connected each other through SH group of cysteine at C-terminus thereof or its vicinity.

14. The immunosuppressive agent of Claim 12 or 13, wherein said cell adhesion protein is a dimer of a protein which has an amino acid sequence deficient in the 1st to the 28th amino acids in the amino acid sequence of SEQ ID NO: 1; and/or
into which glycine-serine-glycine-serine-serine is inserted just before cysteine at the C-terminus; or into which glycine-serine-serine is inserted just before cysteine at the C-terminus, and glycine-proline is inserted just after said cysteine,
wherein two of said proteins are connected each other through SH group of cysteine at C-terminus thereof or its vicinity.

15. The immunosuppressive agent of Claim 12, wherein said cell adhesion protein is a dimer of a protein having the amino acid sequence of SEQ ID NO: 4, or a dimer of a derivative having an amino acid sequence deficient in the 1st to the 28th amino acids in the amino acid sequence of SEQ ID NO: 4, wherein two of said proteins or derivatives are connected each other through SH group of cysteine at C-terminus thereof or its vicinity.

16. The immunosuppressive agent of Claim 12, wherein said cell adhesion protein is a dimer of a protein having the amino acid sequence of SEQ ID NO: 5 or 6, wherein two of said proteins are connected each other through SH group or cysteine at C-terminus thereof or its vicinity.

17. The immunosuppressive agent of Claim 12, 13, 14, 15 or 16, wherein said human cell is a human peripheral blood cell, and said immunosuppressive cell is obtained by culturing said human peripheral blood cell in the presence of 1L-2 and/or GM-CSF together with a cell adhesion protein.

18. The immunosuppressive agent of Claim 12, 13, 14, 15, 16 or 17, wherein said immunosuppressive cell is obtained by culturing a human cell in a medium containing serum.

19. The immunosuppressive agent of Claim 18, wherein said serum is autologous serum.

20. An immunosuppressive agent which comprises as an effective ingredient a cell adhesion protein.
